# EUROPEAN PATENT APPLICATION

(11) **EP 4 647 443 A1**
(43) Date of publication of application: **12.11.2025**
(21) Application number: 24174268.3
(22) Date of filing: 06.05.2024
(51) Int. Cl.: C07K 16/12

(54) **THERAPEUTIC VARIABLE DOMAIN OF HEAVY CHAIN (VHH) ANTIBODY FUSIONS CO-NEUTRALIZING ALPHA- AND GAMMA-HEMOLYSINS FROM STAPHYLOCOCCUS AUREUS**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention pertains to the fields of antibody technology, medicine, pharmacology, infection biology, and medical diagnostics. More specifically, the present disclosure provides VHH antibodies that neutralize cytotoxic gamma-hemolysins of *S*. *aureus,* an approach for co-neutralizing alpha- and gamma-hemolysins by VHH-fusions, and the implementation of this approach.

## Description

### Field of the invention

The present invention pertains to the fields of antibody technology, medicine, pharmacology, infection biology, and medical diagnostics. More specifically, the present disclosure provides VHH antibodies that neutralize cytotoxic gamma-hemolysins of *S*. *aureus,* an approach for co-neutralizing alpha- and gamma-hemolysins by VHH-fusions, and the implementation of this approach.

### Background

*Staphylococcus aureus* is one of the most prevalent bacterial pathogens, causing a wide range of diseases, including superficial skin infections, life-threatening pneumonia, bacteremia, and sepsis. In developed countries, *S*. *aureus* is the most common cause of blood infections. In the US, the number of deaths from *S*. *aureus* was greater than those caused by AIDS, tuberculosis, and viral hepatitis together (Cheung et al., 2021). The lack of an approved vaccine and complicated treatments make *S*. *aureus* a major global health problem.

*S*. *aureus* is a commensal bacterium, with an estimated 30% of the population being carriers (Tong et al., 2015; Saleh et al., 2022). However, it can readily cause infections upon injuries to the skin or mucosal membranes. The human immune system can normally eliminate invading bacteria within minutes, using complement, macrophages, and - after repeated contact - also antibodies. Virulent bacteria, however, use virulence factors to evade the immune response. Virulence factors comprise mostly surface and extracellular secreted proteins, including bacterial toxins, complement-inactivating factors, inhibitors of phagocytosis, or proteases that cleave immunoglobulins or extracellular matrix. Unlike other bacteria, which deploy only one or a few, *S*. *aureus* employs >20 virulence factors to promote its pathogenicity (Cheung et al., 2021).

Antibiotics are key to treating bacterial infections, but the rise of antibiotic-resistant infections makes therapies increasingly unreliable. The frequent occurrence of multi-resistant *S*. *aureus* isolates poses a particular challenge. MRSA (methicillin-resistant *S*. *aureus*) is the most commonly identified antibiotic-resistant pathogen worldwide. Infected patients have longer hospital stays, higher treatment costs, and higher mortality (Ippolito et al., 2010). The first MRSA strain was characterized in 1961, just one year after the introduction of methicillin for clinical treatments (Ippolito et al., 2010; Turner et al., 2019). Since then, a plethora of strains have been characterized that are resistant to an increasing number of antibiotics. Some clinical isolates are resistant to ≥10 antibiotic classes (Saleh et al., 2022). New antibiotics for MRSA and, more generally, for *S*. *aureus* therapy have been developed or are in the pipeline. Currently, vancomycin is an antibiotic of last resort in non-critically ill patients (Cheung et al., 2021). Yet, although not widespread, vancomycin-resistant *S*. *aureus* isolates have emerged and vancomycin treatments are failing more frequently (Lodise et al., 2008; Ye et al., 2020).

Another limitation of antibiotic therapy is that even if the bacteria are killed, it cannot remove already circulating bacterial toxins from the body. Antibiotics may even enhance toxin release. Therefore, there is a need for alternative treatment strategies that target virulence factors. One such "anti-toxin treatment" approach is to use antibodies to neutralize virulence factors, which should stop the toxin effects, "disarm" the pathogen and allow the immune system to clear the infection.

Pore-forming toxins (PFTs) are key virulence factors of *S*. *aureus.* They are secreted as (water-) soluble monomers, bind to specific membrane receptors on target cells, and assemble into 14-40 Å wide, β-barrel transmembrane pores. Such pores cause leakage of ions and other small molecules, destabilize the membranes, and lead to cell lysis. PFTs mainly target leukocytes to evade the host cell defense system, as well as erythrocytes to make the otherwise limiting iron available for bacterial growth (Spaan et al., 2013; Cassat & Skaar, 2013).

*S*. *aureus* deploys five different toxin pores, all of which adopt β-barrel transmembrane structures: an alpha-hemolysin pore, two kinds of gamma-hemolysin pores, and two kinds of leukocidin pores. Alpha-hemolysin (alpha-toxin, Hla) is the best-characterized cytotoxin of *S*. *aureus,* targeting a wide range of host cells including erythrocytes, monocytes, epithelial and endothelial cells (Divyakolu et al., 2019). Hla assembles into homo-heptameric pores.

Gamma-hemolysins (Hlg or gamma-toxins) on the other hand, are bicomponent PFTs with HlgB as the class F (fast-eluting in chromatography columns) component and either HlgA or HlgC as the class S (slowly eluting) component. The S component first binds to a unique receptor and then recruits the F component to form a hetero-octameric complex that rearranges to a β-barrel transmembrane pore (Staali & Colin, 2021). Leukocidins are related to gamma-toxins. LukF and LukS make one leukocidin pair, and LukD and LukE form another one. The structures of these pores are highly conserved, and similar to the heptameric pores of Hla. Indeed, all precursor proteins have a molecular weight of 33 kDa and are structurally almost identical. However, the sequence identity between the individual components is only 25-35%, with class F components being more similar to Hla than the class S components.

Hlgs are present in 99% of *S*. *aureus* isolates (Prevost et al., 1995). HlgA uses human chemokine receptors including the Duffy antigen/receptor for chemokines (DARC) expressed in erythrocytes and endothelial cells (Spaan et al., 2015a), as well as CXCR1 and CXCR2 expressed in granulocytes and macrophages (Spaan et al., 2014). HlgC instead uses C5aR1 (complement C5a receptor), expressed in leukocytes, as the major receptor (Spaan et al., 2015b; Zimmermann-Meisse et al., 2017). Consequently, HIgA/HIgB (HlgAB) and HlgC/HlgB (HlgCB) toxins extend the virulence of *S. aureus* by targeting different receptors.

Nanobodies (VHH antibodies) comprise a novel class of therapeutic proteins based on monovalent, camelid-derived heavy chain-only antibodies. In contrast to monoclonal IgGs, which are manufactured in mammalian cells, nanobodies can also be produced in bacteria or yeast. Apart from their low molecular weight, certain nanobodies proved to be hyper-thermostable and display a particularly high affinity (Güttler et al., 2021).

To effectively treat sepsis caused by *S*. *aureus,* several pore-forming toxins should be neutralized. The most economical approach is to do this with a single molecule, for example by a tandem fusion of different VHH antibodies, each neutralizing a different toxin.

PCT/EP2024/052663, the content of which is herein incorporated by reference, discloses VHH antibodies that specifically bind and neutralize *S*. *aureus* Hla.

It was the objective of this invention to provide VHH antibodies against *S*. *aureus* Hlg components and VHH fusions that effectively co-neutralize alpha- and gamma-hemolysin toxins.

### Summary of the invention

The present disclosure provides single-domain VHH antibodies specifically binding (recognizing) an *S*. *aureus* alpha-hemolysin (Hlg). They recognize and/or prevent membrane-binding and/or oligomerization of and hemolysis by *S*. *aureus* Hlg and are suitable for diagnostic, prophylactic and/or therapeutic applications.

In certain embodiments, the VHH antibody is in monovalent form, e.g., as a single VHH domain or as a fusion to a heterologous protein such as serum albumin useable in a wide range of formats. In certain embodiments, the VHH antibody is in multivalent form, e.g., as bivalent Fc-fusion. In certain embodiments, the VHH antibody is a hetero-multimeric VHH antibody comprising of two or more, e.g. 2, 3 or 4 different VHH antibody units.

The use in a monovalent format is possible due to their very high affinity. In certain embodiments, the VHH antibodies have - in the monovalent format - a target affinity in picomolar or even low picomolar range. In certain embodiments, the VHH antibodies neutralize Hlg with high potency.

A first aspect of the present disclosure is a VHH antibody directed against *S*. *aureus* Hlg as described herein. Preferred VHH antibodies of the present invention, their designations, binding characteristics, i.e., Hlg epitope, Hlg affinity, and Hlg neutralization as well as their thermostabilities are listed in the following Table 1:

**Table 1: Anti-Hlg VHHs, their thermostability, affinities for respective Hlg component (expressed as KD values) and inhibitory properties.**

| **Table 1** | | | | | | | |
|---|---|---|---|---|---|---|---|
| **VHH** | **Class** | **Anti** | **Epitope** | **Thermostability (°C)** | | **Affinity (pM)** | **Toxin inhibition** |
| | | | | **-DTT** | **+DTT** | | |
| Bm29C02 | Bm29C02 | HlgB | 2 | >95 | >95 | 10 | Yes |
| Bm29H04 | Bm29C02 | HlgB | 2 | >95 | >95 | 60 | Yes |
| Bm29E06 | Bm29E06 | HlgB | 2 | 58 | 28 | 30 | Yes |
| Bm29F05 | Bm29E06 | HlgB | 2 | >95 | >95 | 20 | Yes |
| Bm29B04 | Bm29B04 | HlgB | 1 | >95 | >95 | ≤10 | Yes |
| Bm29B05 | Bm29B04 | HlgB | 1 | >95 | 41 | ≤10 | Yes |
| Bm29D07 | | HlgB | 3 | 44 | 43 | ≤10 | No |
| | | | | | | | |
| Bm31D04 | Bm31 G03 | HlgA | 1 | >95 | >95 | 50 | Yes |
| Bm31G03 | Bm31G03 | HlgA | 1 | >95 | >95 | 15 | Yes |
| Bm31G04 | Bm31G03 | HlgA | 1 | >95 | >95 | 25 | Yes |
| Bm31F09 | Bm31G12 | HlgA | 1 | >95 | >95 | 90 | Yes |
| Bm31G12 | Bm31G12 | HlgA | 1 | >95 | >95 | 25 | Yes |
| Bm31A09 | Bm31D02 | HlgA | 1 | >95 | >95 | 50 | Yes |
| Bm31D02 | Bm31D02 | HlgA | 1 | >95 | >95 | 70 | Yes |
| Bm31A04 | | HlgA | 1 | >95 | >95 | 60 | Yes |
| Bm31A08 | | HlgA | 2 | 67 | 67 | ≤10 | No |
| Bm31B04 | | HlgA | 1 | 72 | 62 | ≤10 | Yes |
| | | | | | | | |
| Bm32A03 | Bm32A03 | HlgC | 1 | >95 | 52 | 25 | Yes |
| Bm32B07 | Bm32A03 | HlgC | 1 | >95 | 52 | 45 | Yes |
| Bm32D01 | Bm32A03 | HlgC | 1 | | | 30 | Yes |
| Bm32A08 | Bm32A08 | HlgC | 2 | >95 | >95 | 45 | Yes |
| Bm32F03 | Bm32A08 | HlgC | 2 | >95 | 45 | 850 | No |
| | | | | | | | |

A list of complete VHH sequences of anti-Hlg VHH antibodies is shown at the end of the specification.

A further aspect of the present invention relates to a set of two or more different VHH antibodies, wherein at least one VHH antibody is as described above.

A VHH antibody described above is suitable for use in medicine, e.g., human medicine, particularly for use in therapy, e.g., in the prevention, treatment and/or mitigation of a condition caused by, associated with and/or accompanied by an infection with Hlg-positive *S*. *aureus,* particularly by an infection with a drug-resistant Hlg-positive *S*. *aureus,* or in diagnostics, e.g., for detecting *S*. *aureus* Hlg in a patient sample, e.g., in a body fluid or tissue sample, or in research.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for the recombinant production of a VHH antibody as described above, comprising cultivating a cell or an organism as described above in a suitable medium and obtaining the VHH antibody from the cell or organism or from the medium.

Still a further aspect of the invention relates to a method for the prevention, treatment and/or mitigation of a condition caused by, associated with and/or accompanied by an infection with Hlg-positive *S. aureus,* particularly by an infection with a drug-resistant Hlg-positive *S. aureus,* comprising administering an effective dose of the VHH antibody as described above or the set of at least two different VHH antibodies as described above to a subject in need thereof, particularly to a human subject suffering from a disorder caused by, associated and/or accompanied by an infection with Hlg-positive *S. aureus.*

A non-limitative list of Anti-Hlg VHH antibodies of the present invention and their CDR sequences is shown in the following Table 2:

**Table 2: Anti-Hlg VHHs and their CDR regions. Note that CDRs include here also variable residues that flank the actual CDR loops**

| **Table 2** | | | | | | | |
|---|---|---|---|---|---|---|---|
| VHH | SEQ ID NO. | CDR1 | SEQ ID NO. | CDR2 | SEQ ID NO. | CDR3 | SEQ ID NO. |
| Bm29C02 | 5 | GFTLDDWAIGWF | 6 | GVSCTSSRERSSYYR | 7 | AADPQPYPCSLGQLGRYTTW | 8 |
| Bm29H04 | 9 | GVSLDDFAIGWF | 10 | GVSCSSSRDRSEYYR | 11 | AADPQPYPCSVGSLDRYNSW | 12 |
| Bm29E06 | 13 | GVSLYDFAVGWF | 14 | GVSCSSSRDRSEYYR | 15 | AADSQPYPCSLGSLERYDSW | 16 |
| Bm29F05 | 17 | GVSLYDFAVGWW | 18 | GVSCTSSRDRSEYYR | 19 | AADSQPYPCSLGSLERYDSW | 20 |
| Bm29B04 | 21 | GFTFDDYVIGWF | 22 | AVACISNGDGVANVP | 23 | AAVKERLCVHEGMEYW | 24 |
| Bm29B05 | 25 | GFTFDDYVIGWF | 26 | AVACISNGDGVTNYP | 27 | ATAKERLCVQGGMEYW | 28 |
| Bm29D07 | 29 | SIIVSYYAIGWF | 30 | GVSRITVPAGNLYYA | 31 | AVSYGDSWVDPSYVDHW | 32 |
| | | | | | | | |
| Bm31D04 | 33 | GIIFDFYDMGWY | 34 | LVAGISISGSTTYA | 35 | NAHHVDSDYLRGYDYF | 36 |
| Bm31G03 | 37 | GIIFSFYDMGWY | 38 | VVATISISGRTTYA | 39 | NAQHVDSDYLAGYDYF | 40 |
| Bm31G04 | 41 | GITFTFYDMGWY | 42 | LVATISISSSTTYA | 43 | NAQHVDSDYLAGYDYF | 44 |
| Bm31F09 | 45 | GFTFDDYVMSWV | 46 | WVSSIHTASSDTYYA | 47 | AADKAPLTIATMTRDEYDYW | 48 |
| Bm31G12 | 49 | GFTVDDYVMSWV | 50 | WVSSIHSYSSDTYYA | 51 | AADKDPLTIATMTRYEYDYW | 52 |
| Bm31A09 | 53 | GSTGSIYVMGWF | 54 | LVASITRGEGSANYA | 55 | HADTGLGDYSDYAPHGYKYDYW | 56 |
| Bm31D02 | 57 | GSTGSIYVMGWF | 58 | LVASITRGEGSANYA | 59 | HADTGLGDYSDYAPHGYKYDYW | 60 |
| Bm31A04 | 61 | ESISNIYVMGWF | 62 | LVAAITSSNNTNYA | 63 | NADWGLGGYSDYGEYDYW | 64 |
| Bm31A08 | 65 | GVVWSKYVMSWV | 66 | WVSGIKSDGSDTYYA | 67 | VLEYGTSWPPRVEEYDYW | 68 |
| Bm31B04 | 69 | GSSFSIYGMSWY | 70 | LVALINVGDWTTYA | 71 | KADLSVSGSSFIRTYW | 72 |
| | | | | | | | |
| Bm32A03 | 73 | GSISSIYAMPWF | 74 | FVAAISWHTNLAYYA | 75 | NVPSKEEYDRTSYYPTGGSW | 76 |
| Bm32B07 | 77 | GRISSIYAMPWF | 78 | FVAAISWHTDLAYYA | 79 | NIPSKEEYDRNYYYPTGGSW | 80 |
| Bm32D01 | 81 | GRISSIYAMPWF | 82 | FVAAISWHTDLTYYA | 83 | NVPSKEQYDRTSYYPTGGSW | 84 |
| Bm32A08 | 85 | GDWFIINSMAWF | 86 | QVATISHGGIPRYA | 87 | AAGGLDYGFPDPTPEEFDYW | 88 |
| Bm32F03 | 89 | GDWFIINSMAWF | 90 | QVATISHGGIPRYA | 91 | AAGGLDYGFPDPTPEEFDYW | 92 |

Still a further aspect of the present invention relates to a hetero-multimeric VHH antibody comprising a first VHH antibody unit which recognizes Hlg, particularly an anti-Hlg VHH antibody as herein described above, and a second VHH antibody unit which recognizes Hla, particularly an anti-Hla antibody as described in PCT/EP2024/052663.

In certain embodiments, the hetero-multimeric VHH antibody is a tandem fusion of VHH antibody units comprising a first unit which is an anti-Hlg VHH antibody, and second unit which is an anti-Hla VHH antibody wherein the first unit is covalently fused to the second unit, particularly via a linker. In certain embodiments, the tandem fusion is further fused to a heterologous polypeptide, e.g., an immunoglobulin Fc fragment.

In certain embodiments, the first unit of the hetero-multimeric antibody is an anti-HlgB VHH antibody.

A non-limitative list of anti-Hla VHH antibodies and their CDR sequences is shown in the following Table 3:

**Table 3: Anti-Hla VHH antibodies and their CDR regions. Note that CDRs include here also variable residues that flank the actual CDR loops.**

| **Table 3** | | | | | | | |
|---|---|---|---|---|---|---|---|
| VHH | SEQ ID NO. | CDR1 | SEQ ID NO. | CDR2 | SEQ ID NO. | CDR3 | SEQ ID NO. |
| Ma7A07 | 93 | SGFTLGDYTLG | 94 | SSIASSALSIYIADS | 95 | RGVDSWRWVPSAMDLW | 96 |
| Ma7A08 | 97 | SGFTLGDYTLG | 98 | SSIASSVLSVYIADS | 99 | RGVDSWRWVPSAMDLW | 100 |
| Ma7B03 | 101 | SGFTLSNYYVG | 102 | SSIGSSDLSIYIADS | 103 | RGTDSWRWVPSAMDLW | 104 |
| Ma7B12 | 105 | SGFTLGDYTLG | 106 | SSIASSALSIYIADS | 107 | RGIDNWRWVPSAMDLW | 108 |
| Ma7B01 | 109 | SGRTFSNYAMG | 110 | AVINQIGDSTWYPDF | 111 | ATDPRSYWRIWPHEYGYW | 112 |
| Ma7C02 | 113 | SGRTFSDYAMG | 114 | AVITRSGDSTYYPDS | 115 | ATDPTNYWRIWEHEFDYW | 116 |
| Ma7E01 | 117 | SERTFSNYAMG | 118 | AVINPIGEETWYPDF | 119 | ATDPRSYWRIWEHEFDYW | 120 |
| Ma7F02 | 121 | SGSIDSLHTMG | 122 | AVVSWSGGNTYYPDY | 123 | AESGSGNYWKIWEHEFDSW | 124 |
| Ma7D06 | 125 | SGSISGIDTMA | 126 | AVIKWAAGSTWYPDF | 127 | AAAGEKYYYRLFPYEYDYW | 128 |
| Ma7C11 | 129 | SGSISSVNGMG | 130 | AQVSLLDSSTYYADS | 131 | ATKLRIGTAFSSEYDYW | 132 |
| Ma8E03 | 133 | SGSSLDHYVIG | 134 | SCISRSGGSTNYADS | 135 | AQRNLGNFCVLGWLEYDDW | 136 |
| Ma8G02 | 137 | SGSSLDHYVIG | 138 | SCISRSGGSTNYADS | 139 | AQRNLGNFCVLGWVEYDDW | 140 |
| Ma8H03 | 141 | SGSSLDHYVIG | 142 | SCISSGGSTNYADS | 143 | AQRNLGNFCVLGWLEYDDW | 144 |
| Ma8A05 | 145 | SPASGFTLDLYTI A | 146 | SAISLSDEITYYSDA | 147 | IGAFDFILKEADADYW | 148 |
| Ma8D05 | 149 | SPASGFTLDLYTI A | 150 | SAISLSDEITYYSDT | 151 | IGAFDFILKEADADYW | 152 |
| Ma8D07 | 153 | SESIYKLNAMG | 154 | TTISSGGSTFYTDP | 155 | ANPTYNHYSAR | 156 |
| Ma8D08 | 157 | SESIYKLNAMG | 158 | TTISSGGSTFYTDP | 159 | ANPTYNHYSAR | 160 |
| Bm28F09 | 161 | SGFTSKNYYIG | 162 | ASIGASDGSLYIADS | 163 | TGRDSWRWVPSAMDYW | 164 |
| Bm28H01 | 165 | SGFTLGDRTLG | 166 | ASIASGVLSAYIADS | 167 | RGVDSWRWVPSTMDLW | 168 |
| Ma31A12 | 169 | SAASGFTLDLYTI A | 170 | SAISLSDEITYYSDA | 171 | IGAFDFILKEVDADYW | 172 |
| Ma31B06 | 173 | SPASGFTLDLYTI A | 174 | SAISLSDEITYYSDA | 175 | IGAFDFILKEADADYW | 176 |
| Bm28C04 | 177 | SGRTFNSYALG | 178 | ASIIRSTGGTSYADS | 179 | GGDGHIYAFGYDYW | 180 |
| Bm28H07 | 181 | SGRTFNSYALG | 182 | ASIIRSTGGTSYADS | 183 | GGDGHIYAFGYDYW | 184 |
| Bm28C09 | 185 | SGRNFNSYAMG | 186 | AAIIRSTGKTTYADSDS | 187 | GGDAGPYAFGYDYW | 188 |
| Bm28H12 | 189 | SGRNFNSYAMG | 190 | AAIIRSTGKTTYADSDS | 191 | GGDAGPYAFGYDYW | 192 |
| Bm28A12 | 193 | SGRALSSYNLA | 194 | ATIMPSADKAHYPDF | 195 | REPSYYHALFEYEYNLW | 196 |

### Embodiments of the invention

In the following, specific embodiments of the invention are disclosed as follows:
1. A VHH antibody recognizing an *S. aureus* gamma-hemolysin (Hlg).
2. The VHH antibody of embodiment 1 which recognizes *S. aureus* gamma-hemolysin component A (HlgA), *S. aureus* gamma-hemolysin component B (HlgB), or *S. aureus* gamma-hemolysin component A (HlgC).
3. The VHH antibody of embodiment 1 or 2, which binds to the same epitope on HlgB as the VHH antibody Bm29C02 having a VHH sequence as shown in SEQ. ID NO: 5 and/or the VHH antibody Bm29E06 having a VHH sequence as shown in SEQ. ID NO: 13.
4. The VHH antibody of embodiment 1 or 2, which binds to the same epitope on HlgB as the VHH antibody Bm29B04 having a VHH sequence as shown in SEQ. ID NO: 21.
5. The VHH antibody of embodiment 1 or 2, which binds to the same epitope on HlgA as the VHH antibody Bm31G03 having a VHH sequence as shown in SEQ. ID NO: 37 and/or the VHH antibody Bm31G12 having a VHH sequence as shown in SEQ. ID NO: 49.
6. The VHH antibody of embodiment 1 or 2, which binds to the same epitope on HlgC as the VHH antibody Bm32A03 having a VHH sequence as shown in SEQ. ID NO: 73.
7. The VHH antibody of embodiment 1 or 2, which binds to the same epitope on HlgC as the VHH antibody Bm32A08 having a VHH sequence as shown in SEQ. ID NO: 85.
8. A VHH antibody recognizing an S. *aureus* HlgB polypeptide, comprising
   (a) a CDR3 sequence as shown in SEQ. ID NO: 8, 12, 16, 20, 24, 28, 32, or 36,
   (b) a CDR3 sequence, which has an identity of at least 80%, at least 90% or at least 95% to a CDR3 sequence of (a).
9. A VHH antibody recognizing an *S. aureus* HlgA polypeptide, comprising
   (a) a CDR3 sequence as shown in SEQ. ID NO: 40, 44, 48, 52, 56, 60, 64, 68, or 72,
   (b) a CDR3 sequence, which has an identity of at least 80%, at least 90% or at least 95% to a CDR3 sequence of (a).
10. A VHH antibody recognizing an *S. aureus* HlgC polypeptide, comprising
   (a) a CDR3 sequence as shown in SEQ. ID NO: 76, 80, 84, 88, or 92,
   (b) a CDR3 sequence, which has an identity of at least 80%, at least 90% or at least 95% to a CDR3 sequence of (a).
11. The VHH antibody of any of the preceding embodiments, comprising
   (a) a combination of CDR1, CDR2 and CDR3 sequences as shown in SEQ. ID NO: 6-8, 10-12, 14-16, 18-20, 22-24, 26-28, 30-32, 34-36, 38-40, 42-44, 46-48, 50-52, 54-56, 58-60, 62-64, 66-68, 70-72, 74-76, 78-80, 82-84, 86-88 or 90-92; or
   (b) a combination of CDR1, CDR2 and CDR3 sequences which has an identity of at least 80%, at least 90% or at least 95% to a combination of CDR1, CDR2 and CDR3 sequences of (a).
12. The VHH antibody of any one of the preceding embodiments, comprising
   (a) a VHH sequence as shown in SEQ. ID NO: 5, 9, 13, 17, 21, 25, 29, 33, 37, 41, 45, 49, 53, 57, 61, 65, 69, 73, 77, 81, 85, or 89;
   (b) a sequence, which has an identity of at least 80%, at least 90%, at least 95% or at least 99% to a VHH sequence of (a).
13. The VHH antibody of any one of the preceding embodiments, wherein the binding affinity expressed as dissociation constant KD to HlgC, HlgA or HlgB is about 1 nM or less, about 0.1 nM or less, or about 0.05 nM or less.
14. The VHH antibody of any one of the preceding embodiments, which neutralizes Hlg, particularly by inhibiting Hlg-induced hemolysis of erythrocytes and/or by inhibiting Hlg-induced cytotoxicity.
15. The VHH antibody of any one of the preceding embodiments, which is stable, particularly thermostable, or hyperthermostable.
16. The VHH antibody of embodiment 15, which has a melting temperature of at least about 40°C, of at least about 50°C, of at least about 60°C, of at least 80°C or of at least about 95°C when measured under non-reducing conditions.
17. The VHH antibody of embodiment 15 or 16, which has a melting temperature of at least about 40°C, of at least about 50°C, of at least about 60°C, of at least 80°C or of at least about 95°C when measured under reducing conditions.
18. The VHH antibody of any one of embodiments 1-17, which is selected from VHH antibody Bm29C02 having a VHH sequence as shown in SEQ. ID NO: 5 or a VHH antibody, which is a variant thereof.
19. The VHH antibody of any one of embodiments 1-17, which is selected from VHH antibody Bm29H04 having a VHH sequence as shown in SEQ. ID NO: 9 or a VHH antibody, which is a variant thereof
20. The VHH antibody of any one of embodiments 1-17, which is selected from VHH antibody Bm29E06 having a VHH sequence as shown in SEQ. ID NO: 13 or a VHH antibody, which is a variant thereof
21. The VHH antibody of any one of embodiments 1-17, which is selected from VHH antibody Bm29F05 having a VHH sequence as shown in SEQ. ID NO: 17 or a VHH antibody, which is a variant thereof.
22. The VHH antibody of any one of embodiments 1-17, which is selected from VHH antibody Bm29B04 having a VHH sequence as shown in SEQ. ID NO: 21 or a VHH antibody, which is a variant thereof.
23. The VHH antibody of any one of embodiments 1-17, which is selected from VHH antibody Bm29B05 having a VHH sequence as shown in SEQ. ID NO: 25 or a VHH antibody, which is a variant thereof.
24. The VHH antibody of any one of embodiments 1-17, which is selected from VHH antibody Bm29D07 having a VHH sequence as shown in SEQ. ID NO: 29 or a VHH antibody, which is a variant thereof.
25. The VHH antibody of any one of embodiments 1-17, which is selected from VHH antibody Bm31D04 having a VHH sequence as shown in SEQ. ID NO: 33 or a VHH antibody, which is a variant thereof.
26. The VHH antibody of any one of embodiments 1-17, which is selected from VHH antibody Bm31G03 having a VHH sequence as shown in SEQ. ID NO: 37 or a VHH antibody, which is a variant thereof.
27. The VHH antibody of any one of embodiments 1-17, which is selected from VHH antibody Bm31G04 having a VHH sequence as shown in SEQ. ID NO: 41 or a VHH antibody, which is a variant thereof.
28. The VHH antibody of any one of embodiments 1-17, which is selected from VHH antibody Bm31F09 having a VHH sequence as shown in SEQ. ID NO: 45 or a VHH antibody, which is a variant thereof.
29. The VHH antibody of any one of embodiments 1-17, which is selected from VHH antibody Bm31G12 having a VHH sequence as shown in SEQ. ID NO: 49 or a VHH antibody, which is a variant thereof.
30. The VHH antibody of any one of embodiments 1-17, which is selected from VHH antibody Bm31A09 having a VHH sequence as shown in SEQ. ID NO: 53 or a VHH antibody, which is a variant thereof.
31. The VHH antibody of any one of embodiments 1-17, which is selected from VHH antibody Bm31D02 having a VHH sequence as shown in SEQ. ID NO: 57 or a VHH antibody, which is a variant thereof.
32. The VHH antibody of any one of embodiments 1-17, which is selected from VHH antibody Bm31D04 having a VHH sequence as shown in SEQ. ID NO: 61 or a VHH antibody, which is a variant thereof.
33. The VHH antibody of any one of embodiments 1-17, which is selected from VHH antibody Bm31A08 having a VHH sequence as shown in SEQ. ID NO: 65 or a VHH antibody, which is a variant thereof.
34. The VHH antibody of any one of embodiments 1-17, which is selected from VHH antibody Bm31B04 having a VHH sequence as shown in SEQ. ID NO: 69 or a VHH antibody, which is a variant thereof.
35. The VHH antibody of any one of embodiments 1-17, which is selected from VHH antibody Bm32A03 having a VHH sequence as shown in SEQ. ID NO: 73 or a VHH antibody, which is a variant thereof.
36. The VHH antibody of any one of embodiments 1-17, which is selected from VHH antibody Bm32B07 having a VHH sequence as shown in SEQ. ID NO: 77 or a VHH antibody, which is a variant thereof.
37. The VHH antibody of any one of embodiments 1-17, which is selected from VHH antibody Bm32D01 having a VHH sequence as shown in SEQ. ID NO: 81 or a VHH antibody, which is a variant thereof.
38. The VHH antibody of any one of embodiments 1-17, which is selected from VHH antibody Bm32A08 having a VHH sequence as shown in SEQ. ID NO: 85 or a VHH antibody, which is a variant thereof.
39. The VHH antibody of any one of embodiments 1-17, which is selected from VHH antibody Bm32F03 having a VHH sequence as shown in SEQ. ID NO: 89 or a VHH antibody, which is a variant thereof.
40. The VHH antibody of any one of the preceding embodiments, which is non-glycosylated, or which is glycosylated.
41. The VHH antibody of any one of the preceding embodiments, which is produced in a bacterium, e.g., *E. coli,* or in a yeast, e.g., *Pichia pastoris,* or in a human or animal cell, e.g., an insect cell or a mammalian cell, e.g., a CHO cell.
42. The VHH antibody of any one of the preceding embodiments, which is in a monovalent format.
43.The VHH antibody of any one of embodiments 1-41, which is in a multimeric format.
44. The VHH antibody of embodiment 43, which is in a dimeric format.
45. The VHH antibody of embodiment 44, which is in a homodimeric or in a heterodimeric format.
46. The VHH antibody of any one of the preceding embodiments, which is covalently or non-covalently conjugated to a heterologous moiety, e.g., a labeling group, a capture group, or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
47. The VHH antibody of any one of the preceding embodiments, which is fused to a heterologous polypeptide moiety, or which is fused to an Fc Fragment, or which is fused to serum albumin or an albumin-binding moiety.
48. The VHH antibody of any one of the preceding embodiments, which is conjugated to one or several non-peptidic polymer moieties, preferably hydrophilic polymer moieties, such as polyethylene glycol (PEG).
49. A hetero-multimeric VHH antibody comprising of two or more different VHH antibody units, e.g., 2, 3 or 4 different VHH antibody units wherein at least one VHH antibody unit comprises a VHH antibody of any of embodiments 1-48.
50. The hetero-multimeric VHH antibody of embodiment 49, which is a tandem fusion of VHH antibody units comprising a first anti-Hlg VHH antibody unit, and a second VHH antibody wherein the first unit is covalently fused to the second unit.
51. The hetero-multimeric VHH antibody of embodiment 49 or 50, which comprises at least two VHH antibody units recognizing different Hlg polypeptides, particularly selected from HlgA, HlgB, and HlgC.
52. The hetero-multimeric VHH antibody of embodiment 49 or 50, which comprises at least two VHH antibody units recognizing different epitopes on the same Hlg polypeptide, particularly different epitopes on HlgA, HlgB, or HlgC.
53. The hetero-multimeric VHH antibody of embodiment 49 or 50, which comprises a first VHH antibody unit which recognizes Hlg and a second VHH antibody unit which recognizes Hla.
54. The hetero-multimeric VHH antibody of any one of embodiments 49-53 wherein the VHH antibody units are covalently fused to each other via a linker.
55. The hetero-multimeric VHH antibody of embodiment 54 wherein the linker has a length of 5-50 amino acids, particularly a length of 10-30 amino acids.
56. The hetero-multimeric VHH antibody of embodiment 54 or 55 wherein the linker substantially consists of amino acids G, E, S and T.
57. The hetero-multimeric VHH antibody of any one of embodiments 49-56 which further comprises a heterologous polypeptide, e.g., an immunoglobulin Fc fragment.
58.The hetero-multimeric VHH antibody of embodiment 57 wherein the heterologous polypeptide is at the C-terminus of the heteromultimeric VHH antibody.
59. The hetero-multimeric VHH antibody of any one of embodiments 49-58 wherein the anti-Hlg VHH antibody comprises
   (a) a combination of CDR1, CDR2 and CDR3 sequences as shown in SEQ. ID NO: 6-8, 10-12, 14-16, 18-20, 22-24, 26-28, 30-32, 34-36, 38-40, 42-44, 46-48, 50-52, 54-56, 58-60, 62-64, 66-68, 70-72, 74-76, 78-80, 82-84, 86-88 or 90-92; or
   (b) a combination of CDR1, CDR2 and CDR3 sequences which has an identity of at least 80%, at least 90% or at least 95% to a combination of CDR1, CDR2 and CDR3 sequences of (a).
60. The hetero-multimeric VHH antibody of any one of embodiments 49-59 wherein the anti-Hlg VHH antibody comprises
   (a) a VHH sequence as shown in SEQ. ID NO: 5, 9, 13, 17, 21, 25, 29, 33, 37, 41, 45, 49, 53, 57, 61, 65, 69, 73, 77, 81, 85, or 89;
   (b) a sequence, which has an identity of at least 80%, at least 90%, at least 95% or at least 99% to a VHH sequence of (a).
61. The hetero-multimeric VHH antibody of any one of embodiments 49-60 wherein the anti-Hlg VHH antibody recognizes HlgB.
62. The hetero-multimeric VHH antibody of any one of embodiments 49-61 comprising the anti-Hlg VHH antibody Bm29C02 having a VHH sequence as shown in SEQ. ID NO: 5 or a VHH antibody, which is a variant thereof.
63. The hetero-multimeric VHH antibody of any one of embodiments 53-62 wherein the anti-Hla VHH antibody comprises
   (a) a combination of CDR1, CDR2 and CDR3 sequences as shown in SEQ. ID NO: 94-96, 98-100, 102-104, 106-108, 110-112, 114-116, 118-120, 122-124, 126-128, 130-132, 134-136, 138-140, 142-144, 146-148, 150-152, 154-156, 158-160, 162-164, 166-168, 170-172, 174-176, 178-180, 182-184, 186-188, 190-192 or 194-196; or
   (b) a combination of CDR1, CDR2 and CDR3 sequences which has an identity of at least 80%, at least 90% or at least 95% to a combination of CDR1, CDR2 and CDR3 sequences of (a).
64. The hetero-multimeric VHH antibody of any one of embodiments 53-63 wherein the anti-Hla VHH antibody comprises
   (c) a VHH sequence as shown in SEQ. ID NO: 93, 97, 101, 105, 109, 113, 117, 121, 125, 129, 133, 137, 141, 145, 149, 153, 157, 161, 165, 169, 173, 177, 181, 185, 189 or 193;
   (d) a sequence, which has an identity of at least 80%, at least 90%, at least 95% or at least 99% to a VHH sequence of (a).
65. The hetero-multimeric VHH antibody of any one of embodiments 53-64 comprising the anti-Hla VHH antibody Ma7A07 having a VHH sequence as shown in SEQ. ID NO: 93 or a VHH antibody, which is a variant thereof.
66. The hetero-multimeric VHH antibody of any one of embodiments 53-65 comprising
   (i) the anti-Hlg VHH antibody Bm29C02 having a VHH sequence as shown in SEQ. ID NO: 5 or a VHH antibody, which is a variant thereof, and
   (ii) the anti-Hla VHH antibody Ma7A07 having a VHH sequence as shown in SEQ. ID NO: 93 or a VHH antibody, which is a variant thereof.
67. A set of two or more different VHH antibodies comprising at least one VHH antibody of any one of embodiments 1-66.
68. The set of embodiment 67 comprising
   i) the anti-Hlg VHH antibody Bm29C02 having a VHH sequence as shown in SEQ. ID NO: 5 or a VHH antibody, which is a variant thereof, and
   (ii) the anti-Hla VHH antibody Ma7A07 having a VHH sequence as shown in SEQ. ID NO: 93 or a VHH antibody, which is a variant
69. The VHH antibody of any one of embodiments 1-66 or the set of any one of embodiments 67-68 for use in medicine, particularly for use in therapy or diagnostics.
70. The VHH antibody of any one of embodiments 1-66 or the set of any one of embodiments 67-68 for use in the prevention, treatment and/or mitigation of a condition caused by, associated with and/or accompanied by an infection with an Hlg-positive and optionally Hla-positive *S. aureus.*
71. The VHH antibody of any one of embodiments 1-66 or the set of any one of embodiments 67-68 for the use of any one of embodiments 69-70 wherein the *S. aureus* is a drug-resistant Hlg-positive and optionally Hla-positive *S. aureus.*
72. The VHH antibody of any one of embodiments 1-66 or the set of any one of embodiments 67-68 for the use of any one of embodiments 69-71, wherein the condition is selected from a skin infection, pneumonia, bacteremia, sepsis, meningitis, osteomyelitis, and/or endocarditis.
73. The VHH antibody of any one of embodiments 1-66 or the set of any one of embodiments 67-68 for the use of any one of embodiments 69-72 in a human subject.
74. The VHH antibody of any one of embodiments 1-66 or the set of any one of embodiments 67-68 for the use of any one of embodiments 69-73, which is administered locally.
75. The VHH antibody of any one of embodiments 1-66 or the set of any one of embodiments 67-68 for the use of any one of embodiments 69-74, which is administered systemically.
76. The VHH antibody of any one of embodiments 1-66 or the set of any one of embodiments 67-68 for the use of any one of embodiments 69-75, which is administered topically, parenterally, e.g., intravenously, pulmonally, e.g., by inhalation, or nasally, e.g., by spraying.
77. The VHH antibody of any one of embodiments 1-66 or the set of any one of embodiments 67-68 for the use of any one of embodiments 69-76, which is administered as a monotherapy.
78. The VHH antibody of any one of embodiments 1-66 or the set of any one of embodiments 67-68 for the use of any one of embodiments 69-76, which is administered sequentially and/or simultaneously as a combination therapy with at least one further active agent.
79. The VHH antibody of any one of embodiments 1-66 or the set of any one of embodiments 67-68 for the use of any one of embodiments 69-76 or 78, which is administered in combination with an antibiotic against *S. aureus,* e.g., vancomycin, and optionally with a further antibiotic against a different bacterium.
80. A pharmaceutical composition comprising as an active agent a VHH antibody of any one of embodiments 1-66 or the set of any one of embodiments 67-68, and a pharmaceutically acceptable carrier.
81.A nucleic acid molecule encoding a VHH antibody or a VHH antibody containing unit according to any one of embodiments 1-66, preferably in operative linkage with a heterologous expression control sequence.
82. A vector comprising a nucleic acid molecule according to embodiment 81.
83. A recombinant cell or non-human organism transformed or transfected with a nucleic acid molecule according to embodiment 81 or a vector according to embodiment 82.
84. The cell or organism of embodiment 83, which is selected from a bacterium such as *E*. *coli Bacillus sp.,* a unicellular eukaryotic organism, e.g., yeast such as *Pichia pastoris,* or *Leishmania,* an insect cell, a mammalian cell, or a plant cell.
85. A method for recombinant production of a VHH antibody of any one of embodiments 1-66, comprising cultivating a cell or an organism of embodiment 83 or 84 in a suitable medium and obtaining the VHH antibody from the cell or organism or from the medium.
86. The method of embodiment 85, comprising cultivating a yeast such as *Pichia pastoris* and obtaining the VHH antibody from the medium.
87. Use of the VHH antibody of any one of embodiments 1-66 or the set of any one of embodiments 67-68 for detecting Hlg and optionally Hla in a sample.
88. The use of embodiment 87, wherein the sample is a biological sample, e.g., a body fluid such as saliva, sputum, a lung lavage, a swab, blood, serum or plasma, a stool sample, a tissue sample, or a biopsy sample.
89. The use of embodiment 87or 88, wherein the detection comprises the binding of at least 2 different VHH antibodies to an Hlg molecule.
90. The use of embodiment 87, wherein the detection comprises the binding of at least 2 different VHH antibodies, e.g., 2, 3, or 4 VHH antibodies each directed against a different epitope, to an Hlg molecule.
91.The use of any one of embodiments 87-90, wherein the detection comprises a sandwich assay, e.g., a sandwich ELISA.
92. The use of any one of embodiments 87-91, wherein the detection comprises a prior Hlg and optionally Hla enrichment step.
93. A method for the prevention, treatment and/or mitigation of a condition caused by, associated with and/or accompanied by an infection with an Hlg-positive and optionally Hla-positive *S. aureus* comprising administering an effective dose of the VHH antibody of any one of embodiments 1-66 or the set of any one of embodiments 67-68 or the pharmaceutical composition of embodiment 80 to a subject in need thereof, particularly to a human subject.

### Description of the Invention

### VHH antibodies

The present invention relates to a VHH antibody recognizing an *S. aureus* gamma-hemolysin (Hlg).

The VHH antibody may be a monovalent heavy chain-only antibody comprising a CDR1 domain, a CDR2 domain and a CDR3 domain linked by framework regions including, but not being limited to, whole VHH antibodies, e.g., native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The VHH antibody of the present invention may be glycosylated or non-glycosylated.

There are several methods known in the art for determining the CDR sequences of a given antibody molecule, but there is no standard unequivocal method. Determination of CDR sequences from antibody heavy and light chain variable regions can be made according to any method known in the art, including, but not limited to, the methods known as Kabat, Chothia, and IMGT. A selected set of CDRs may include sequences identified by more than one method, namely, some CDR sequences may be determined using Kabat and some using IMGT, for example. According to some embodiments of the present invention, the CDR sequences of a VHH variable region are determined using the Kabat method. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell, e.g., a CHO cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing.

According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which binds to the same epitope of an *S. aureus* Hlg polypeptide as a specific VHH antibody disclosed herein. In certain embodiments, a competing VHH antibody binds the same or an overlapping epitope on the *S. aureus* Hlg polypeptide. For example, the invention refers to a VHH antibody, which binds to the same epitope as a reference antibody, e.g., VHH antibody Bm29C02 or Bm29E06 (binding to epitope 2 on HlgB), VHH antibody Bm29B04 (binding to epitope 1 on HlgB), VHH antibody Bm31G03 or Bm31G12 (binding to epitope 1 on HlgA), VHH antibody Bm32A03 (binding to epitope 1 on HlgC) or VHH antibody Bm32A08 (binding to epitope 2 on HlgC). Binding to the same epitope may be determined by competition measurements, e.g., by label-free biolayer interferometry (BLI) performed as a cross-competition or epitope binning assay using a label-free detection system, e.g., an Octet^{®} system from Sartorius, according to the manufacturer's instructions.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Bm29C02 having a VHH sequence as shown in SEQ. ID NO: 5 or a VHH antibody, which is a variant thereof. In certain embodiments, 1, 2, 3, 4, 5, 6, 7, 8, 9 or 10 amino acids of SEQ. ID NO: 5 are replaced by another amino acid.

In further particular embodiments, the VHH antibody is selected from antibody Bm29H04 having a VHH sequence as shown in SEQ. ID NO: 9 or a VHH antibody, which is a variant thereof. In certain embodiments, 1, 2, 3, 4, 5, 6, 7, 8, 9 or 10 amino acids of SEQ. ID NO: 9 are replaced by another amino acid.

In further particular embodiments, the VHH antibody is selected from antibody Bm29E06 having a VHH sequence as shown in SEQ. ID NO: 13 or a VHH antibody, which is a variant thereof. In certain embodiments, 1, 2, 3, 4, 5, 6, 7, 8, 9 or 10 amino acids of SEQ. ID NO: 13 are replaced by another amino acid.

In further particular embodiments, the VHH antibody is selected from antibody Bm29F05 having a VHH sequence as shown in SEQ. ID NO: 17 or a VHH antibody, which is a variant thereof. In certain embodiments, 1, 2, 3, 4, 5, 6, 7, 8, 9 or 10 amino acids of SEQ. ID NO: 17 are replaced by another amino acid.

In further particular embodiments, the VHH antibody is selected from antibody Bm29B04 having a VHH sequence as shown in SEQ. ID NO: 21 or a VHH antibody, which is a variant thereof. In certain embodiments, 1, 2, 3, 4, 5, 6, 7, 8, 9 or 10 amino acids of SEQ. ID NO: 21 are replaced by another amino acid.

In further particular embodiments, the VHH antibody is selected from antibody Bm29B05 having a VHH sequence as shown in SEQ. ID NO: 25 or a VHH antibody, which is a variant thereof. In certain embodiments, 1, 2, 3, 4, 5, 6, 7, 8, 9 or 10 amino acids of SEQ. ID NO: 25 are replaced by another amino acid.

In further particular embodiments, the VHH antibody is selected from antibody Bm29D07 having a VHH sequence as shown in SEQ. ID NO: 29 or a VHH antibody, which is a variant thereof. In certain embodiments, 1, 2, 3, 4, 5, 6, 7, 8, 9 or 10 amino acids of SEQ. ID NO: 29 are replaced by another amino acid.

In further particular embodiments, the VHH antibody is selected from antibody Bm31D04 having a VHH sequence as shown in SEQ. ID NO: 33 or a VHH antibody, which is a variant thereof. In certain embodiments, 1, 2, 3, 4, 5, 6, 7, 8, 9 or 10 amino acids of SEQ. ID NO: 33 are replaced by another amino acid.

In further particular embodiments, the VHH antibody is selected from antibody Bm31G03 having a VHH sequence as shown in SEQ. ID NO: 37 or a VHH antibody, which is a variant thereof. In certain embodiments, 1, 2, 3, 4, 5, 6, 7, 8, 9 or 10 amino acids of SEQ. ID NO: 37 are replaced by another amino acid.

In further particular embodiments, the VHH antibody is selected from antibody Bm31G04 having a VHH sequence as shown in SEQ. ID NO: 41 or a VHH antibody, which is a variant thereof. In certain embodiments, 1, 2, 3, 4, 5, 6, 7, 8, 9 or 10 amino acids of SEQ. ID NO: 41 are replaced by another amino acid.

In further particular embodiments, the VHH antibody is selected from antibody Bm31F09 having a VHH sequence as shown in SEQ. ID NO: 45 or a VHH antibody, which is a variant thereof. In certain embodiments, 1, 2, 3, 4, 5, 6, 7, 8, 9 or 10 amino acids of SEQ. ID NO: 45 are replaced by another amino acid.

In further particular embodiments, the VHH antibody is selected from antibody Bm31G12 having a VHH sequence as shown in SEQ. ID NO: 49 or a VHH antibody, which is a variant thereof. In certain embodiments, 1, 2, 3, 4, 5, 6, 7, 8, 9 or 10 amino acids of SEQ. ID NO: 49 are replaced by another amino acid.

In further particular embodiments, the VHH antibody is selected from antibody Bm31A09 having a VHH sequence as shown in SEQ. ID NO: 53 or a VHH antibody, which is a variant thereof. In certain embodiments, 1, 2, 3, 4, 5, 6, 7, 8, 9 or 10 amino acids of SEQ. ID NO: 53 are replaced by another amino acid.

In further particular embodiments, the VHH antibody is selected from antibody Bm31D02 having a VHH sequence as shown in SEQ. ID NO: 57 or a VHH antibody, which is a variant thereof. In certain embodiments, 1, 2, 3, 4, 5, 6, 7, 8, 9 or 10 amino acids of SEQ. ID NO: 57 are replaced by another amino acid.

In further particular embodiments, the VHH antibody is selected from antibody Bm31A04 having a VHH sequence as shown in SEQ. ID NO: 61 or a VHH antibody, which is a variant thereof. In certain embodiments, 1, 2, 3, 4, 5, 6, 7, 8, 9 or 10 amino acids of SEQ. ID NO: 61 are replaced by another amino acid.

In further particular embodiments, the VHH antibody is selected from antibody Bm31A08 having a VHH sequence as shown in SEQ. ID NO: 65 or a VHH antibody, which is a variant thereof. In certain embodiments, 1, 2, 3, 4, 5, 6, 7, 8, 9 or 10 amino acids of SEQ. ID NO: 65 are replaced by another amino acid.

In further particular embodiments, the VHH antibody is selected from antibody Bm31B04 having a VHH sequence as shown in SEQ. ID NO: 69 or a VHH antibody, which is a variant thereof. In certain embodiments, 1, 2, 3, 4, 5, 6, 7, 8, 9 or 10 amino acids of SEQ. ID NO: 69 are replaced by another amino acid.

In further particular embodiments, the VHH antibody is selected from antibody Bm32A03 having a VHH sequence as shown in SEQ. ID NO: 73 or a VHH antibody, which is a variant thereof. In certain embodiments, 1, 2, 3, 4, 5, 6, 7, 8, 9 or 10 amino acids of SEQ. ID NO: 73 are replaced by another amino acid.

In further particular embodiments, the VHH antibody is selected from antibody Bm32B07 having a VHH sequence as shown in SEQ. ID NO: 77 or a VHH antibody, which is a variant thereof. In certain embodiments, 1, 2, 3, 4, 5, 6, 7, 8, 9 or 10 amino acids of SEQ. ID NO: 77 are replaced by another amino acid.

In further particular embodiments, the VHH antibody is selected from antibody Bm32D01 having a VHH sequence as shown in SEQ. ID NO: 81 or a VHH antibody, which is a variant thereof. In certain embodiments, 1, 2, 3, 4, 5, 6, 7, 8, 9 or 10 amino acids of SEQ. ID NO: 81 are replaced by another amino acid.

In further particular embodiments, the VHH antibody is selected from antibody Bm32A08 having a VHH sequence as shown in SEQ. ID NO: 85 or a VHH antibody, which is a variant thereof. In certain embodiments, 1, 2, 3, 4, 5, 6, 7, 8, 9 or 10 amino acids of SEQ. ID NO: 85 are replaced by another amino acid.

In further particular embodiments, the VHH antibody is selected from antibody Bm32F03 having a VHH sequence as shown in SEQ. ID NO: 89 or a VHH antibody, which is a variant thereof. In certain embodiments, 1, 2, 3, 4, 5, 6, 7, 8, 9 or 10 amino acids of SEQ. ID NO: 89 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an *E*. *coli* or a *Bacillus sp.* cell, a yeast cell, particularly a *Pichia* yeast cell, an insect cell, e.g., an Sf-9 cell, or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to S. aureus Hlg

The VHH antibodies of the present invention bind to an *S. aureus* Hlg polypeptide, i.e., HlgA, HlgB or HlgC. The inventors have identified VHH antibodies, which bind with high affinity to different epitopes on *S. aureus* HlgA, HlgB or HlgC, as shown in Table 1, supra.

In the context of the present disclosure the term *"S. aureus* Hlg" encompasses *S. aureus* Hlg from different strains, e.g., MRSA-resistant strains, such as USA300, USA400 (CC1), USA500 (CC8) or ST80. These and other suitable strains are e.g., described by Diep et al. (2006); Cortes et al. (2017); Earls et al. (2019); Frisch et al. (2018) and Mairi et al. (2020), the contents of which are herein incorporated by reference.

It should be noted, however, that the term *"S. aureus* Hlg" also encompasses naturally occurring variants of *S. aureus* Hlg polypeptides and genetically modified constructs as described herein.

The inventors have performed their selection and crystallization experiments with HlgA shown in SEQ ID NO: 1, HlgB shown in SEQ ID NO: 2 and HlgC shown in SEQ ID NO: 3.

Further, the inventors have performed experiments with Hla shown in SEQ ID NO: 4 when determining the binding characteristics of tandem fusions directed to both Hlg and Hla.

In certain embodiments, the VHH antibody of the present invention binds to a *S. aureus* Hlg polypeptide, i.e., HlgA, HlgB or HlgC, wherein the binding affinity expressed as dissociation constant K_{D} is about 1 nM or less, of about 0.1 nM or less, or of about 0.05 nM or less. The binding affinity may be determined as described herein in detail in the Examples using the polypeptides of SEQ. ID NO: 1, 2 and 3 as described above.

### Hlg neutralization

The VHH antibodies of the present invention are capable of neutralizing *S. aureus* Hlg, particularly by inhibiting the hemolysis of blood cells, e.g., erythrocytes, and/or by inhibiting cytotoxic activity against HeLa cells as shown in Table 1, supra.

The VHH antibodies of the present invention may alter the biological properties of Hlg components or Hlg toxins and optionally Hla toxins and/or inhibit receptor or membrane binding of Hlg components or Hlg toxins and optionally Hla toxins and/or formation of oligomeric pores

In certain embodiments, the VHH antibody of the present invention inhibits HlgAB-mediated hemolysis of red blood cells. In particular embodiments, the VHH antibody recognizes HlgA, more particularly epitope 1 of HlgA, such as Bm31A04, Bm31A09, Bm31D04, Bm31G03 and Bm31G04. In further particular embodiments, the VHH antibody recognizes HlgB, more particularly epitope 1 or 2 of HlgB such as Bm29C02, Bm29H04, Bm29E06, Bm29F05, Bm29B04, or Bm29B05.

In certain embodiments, the VHH antibody of the present invention inhibits HlgBC-mediated hemolysis of red blood cells. In particular embodiments, the VHH antibody recognizes HlgB, more particularly epitope 1 or 2 of HlgB such as Bm29C02, Bm29H04, Bm29E06, Bm29F05, Bm29B04, or Bm29B05.

In certain embodiments, the VHH antibody of the present invention inhibits HlgBC-mediated cytotoxicity against HeLa cells. In particular embodiments, the VHH antibody recognizes HlgC, more particularly epitope 1 or 2 of HlgC, such as Bm32A03, Bm32B07, and Bm32A08. In further particular embodiments, the VHH antibody recognizes HlgB, more particularly epitope 1 or 2 of HlgB such as Bm29C02, Bm29H04, Bm29E06, Bm29F05, Bm29B04, or Bm29B05

### Stability

For the intended therapeutic application, the anti-Hlg antibodies should not only be highly potent in Hlg neutralization, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable, or hyperthermostable. Preferably, the VHH antibody has a melting point (melting temperature) of at least about 40°C, of at least about 50°C, of at least about 60°C, of at least about 80°C, of at least 90°C or of at least about 95°C, when measured under non-reducing and/or reducing conditions, e.g., in the absence of a reducing agent or the presence of a reducing agent such as dithiothreitol (DTT). Melting temperatures are determined as described herein.

### Sets of VHH antibodies

In a further aspect, the present invention relates to a set comprising at least 2, 3, 4 or more different VHH antibodies wherein the set comprises at least one VHH antibody as described above. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody, e.g., as described above. The set of the present invention may be free from other VHH antibodies.

In particular embodiments, the set of VHH antibodies comprises at least two, e.g., two, three or four complementary VHH antibodies, i.e., VHH antibodies directed against different Hlg polypeptides, directed against different epitopes of a Hlg polypeptide and/or directed against different *S. aureus* toxins or virulence factors .

In certain embodiments, the set of VHH antibodies comprises complementary VHH antibodies directed against different Hlg polypeptides, e.g., at least one VHH antibody against HlgA and at least one VHH antibody against HlgB, at least one VHH antibody against HlgB and at least one VHH antibody against HlgC, or at least one VHH antibody against HlgA, at least one VHH antibody against HlgB and at least one VHH antibody against HlgC.

In certain embodiments, the set of VHH antibodies comprises complementary VHH antibodies directed against different epitopes on a Hlg polypeptide, e.g., at least one VHH antibody against epitope 1 of HlgB and at least one VHH antibody against epitope 2 of HlgB, or at least one VHH antibody against epitope 1 of HlgC and at least one VHH antibody against epitope 2 of HlgC.

In further embodiments, the set of VHH antibodies comprises complementary VHH antibodies directed against different toxins or virulence factors. of *S. aureus,* e.g., pore-forming toxins of *S. aureus.* For example, the set of VHH antibodies may comprise at least one VHH antibody against a Hlg polypeptide as described herein, e.g., a HlgB polypeptide, and at least one VHH antibody against another pore-forming toxin. In certain embodiments, the set comprises at least one VHH antibody against *S. aureus* Hla, particularly a VHH antibody described in PCT/EP2024/052663, or at least one VHH antibody against Leukocidin S (LukS), Leukocidin F (LukF), Leukocidin E (LukE) or Leukocidin D (LukD).

In particular embodiments, the anti-Hla VHH antibody may comprise
(a) a combination of CDR1, CDR2 and CDR3 sequences as shown in SEQ. ID NO: 94-96, 98-100, 102-104, 106-108, 110-112, 114-116, 118-120, 122-124, 126-128, 130-132, 134-136, 138-140, 142-144, 146-148, 150-152, 154-156, 158-160, 162-164, 166-168, 170-172, 174-176, 178-180, 182-184, 186-188, 190-192 or 194-196; or
(b) a combination of CDR1, CDR2 and CDR3 sequences which has an identity of at least 80%, at least 90% or at least 95% to a combination of CDR1, CDR2 and CDR3 sequences of (a).

In further particular embodiments, the anti-Hla VHH antibody may comprise
(a) a VHH sequence as shown in SEQ. ID NO: 93, 97, 101, 105, 109, 113, 117, 121, 125, 129, 133, 137, 141, 145, 149, 153, 157, 161, 165, 169, 173, 177, 181, 185, 189 or 193; or
(b) a sequence, which has an identity of at least 80%, at least 90%, at least 95% or at least 99% to a VHH sequence of (a).

Sets of different VHH antibodies are useful for therapeutic and diagnostic applications as described herein in detail below.

In certain embodiments, a set of VHH antibodies comprises:
(i) the anti-HlgB VHH antibody Bm29C02 having a VHH sequence as shown in SEQ. ID NO: 5 or a VHH antibody, which is a variant thereof, and
(ii) the anti-Hla VHH antibody Ma7A07 having a VHH sequence as shown in SEQ. ID NO: 93 or a VHH antibody, which is a variant thereof.

In certain embodiments, a set of VHH antibodies comprises:
(i) the anti-HlgB VHH antibody Bm29C02 having a VHH sequence as shown in SEQ. ID NO: 5 or a VHH antibody, which is a variant thereof, and
(ii) the anti-Hla VHH antibody Ma7A07 having a VHH sequence as shown in SEQ. ID NO: 93 or a VHH antibody, which is a variant thereof.

In certain embodiments, a set of VHH antibodies comprises:
(i) the anti-HlgB VHH antibody Bm29B04 having a VHH sequence as shown in SEQ. ID NO: 9 or a VHH antibody, which is a variant thereof, and
(ii) the anti-Hla VHH antibody Ma31A12 having a VHH sequence as shown in SEQ. ID NO: 169 or a VHH antibody, which is a variant thereof, and/or
(iii) the anti-Hla VHH antibody Ma8E03 having a VHH sequence as shown in SEQ. ID NO: 133 or a VHH antibody, which is a variant thereof.

In certain embodiments, a set of VHH antibodies comprises:
(i) the anti-HlgB VHH antibody Bm29C02 having a VHH sequence as shown in SEQ. ID NO: 5 or a VHH antibody, which is a variant thereof,
(ii) the anti-Hla VHH antibody Ma7A07 having a VHH sequence as shown in SEQ. ID NO: 93 or a VHH antibody, which is a variant thereof,
(iii) the anti-HlgB VHH antibody Bm29B04 having a VHH sequence as shown in SEQ. ID NO: 9 or a VHH antibody, which is a variant thereof, and
(Iv) the anti-Hla VHH antibody Ma31A12 having a VHH sequence as shown in SEQ. ID NO: 169 or a VHH antibody, which is a variant thereof.

In certain embodiments, a set of VHH antibodies comprises:
(i) the anti-HlgB VHH antibody Bm29C02 having a VHH sequence as shown in SEQ. ID NO: 5 or a VHH antibody, which is a variant thereof,
(ii) the anti-Hla VHH antibody Ma7A07 having a VHH sequence as shown in SEQ. ID NO: 93 or a VHH antibody, which is a variant thereof,
(iii) the anti-HlgB VHH antibody Bm29B04 having a VHH sequence as shown in SEQ. ID NO: 9 or a VHH antibody, which is a variant thereof, and
(iv) the anti-Hla VHH antibody Ma8E03 having a VHH sequence as shown in SEQ. ID NO: 133 or a VHH antibody, which is a variant thereof.

In certain embodiments, a set of VHH antibodies comprises at least one hetero-multimeric VHH antibody comprising two or more different VHH antibody units, e.g.:
(i) a hetero-multimeric VHH antibody comprising the anti-HlgB VHH antibody Bm29C02 having a VHH sequence as shown in SEQ. ID NO: 5 or a VHH antibody, which is a variant thereof, and the anti-Hla VHH antibody Ma7A07 having a VHH sequence as shown in SEQ. ID NO: 93 or a VHH antibody, which is a variant thereof;
(ii) a hetero-multimeric VHH antibody comprising the anti-HlgB VHH antibody Bm29B04 having a VHH sequence as shown in SEQ. ID NO: 9 or a VHH antibody, which is a variant thereof, and the anti-Hla VHH antibody Ma31A12 having a VHH sequence as shown in SEQ. ID NO: 169 or a VHH antibody, which is a variant thereof; and/or
(ii) a hetero-multimeric VHH antibody comprising the anti-HlgB VHH antibody Bm29B04 having a VHH sequence as shown in SEQ. ID NO: 9 or a VHH antibody, which is a variant thereof, and the anti-Hla VHH antibody Ma8E03 having a VHH sequence as shown in SEQ. ID NO: 133 or a VHH antibody, which is a variant thereof.

For example, a set may comprise the heteromultimeric VHH antibody Bm29C02/Ma7A07, the monomeric VHH antibody Bm29B04 and at least one of the monomeric VHH antibodies Ma31A12 and Ma8E03.

Further, a set may comprise at least one of the heteromultimeric VHH antibodies Bm29B04/ Ma31A12 and Bm29B04/Ma8E03, the monomeric VHH antibody Bm29C02 and the monomeric VHH antibody Ma7A07.

Further, a set may comprise at least one of the heteromultimeric VHH antibodies Bm29B04/ Ma31 A12 and Bm29B04/ Ma8E03, and the heteromultimeric VHH antibody Bm29C02/Ma7A07.

### Monovalent and multivalent VHH antibodies

In certain embodiments, the VHH antibody of the present invention is in a monovalent format, i.e., it has a single binding site for an *S. aureus* polypeptide. In these embodiments, the VHH antibody may be present as such or covalently or non-covalently attached to a heterologous moiety, e.g., a peptidic or non-peptidic moiety.

In further embodiments, the VHH antibody of the present invention is in in a multimeric, e.g., dimeric, trimeric or tetrameric format. In these embodiments, several VHH antibody units may be covalently or non-covalently attached to each other together via a linker and/or a multimerization, e.g., dimerization, trimerization or tetramerization moiety. In these embodiments, the VHH antibody of the present invention may be a homo-multimeric or hetero-multimeric antibody.

In certain embodiments, the VHH antibody is a homodimeric VHH antibody, wherein a VHH antibody unit is covalently attached to a dimerization moiety, e.g., an immunoglobulin IgG Fc fragment.

In certain embodiments, the VHH antibody is a heterodimeric VHH antibody, particularly a covalently linked VHH heterodimer comprising a first VHH antibody unit and a second VHH antibody unit wherein the first VHH antibody unit and the second VHH antibody unit bind to different antigens or different epitopes on the same antigen.

In certain embodiments, the multimeric VHH antibody is a hetero-multimeric VHH antibody comprising two or more, e.g., 2, 3 or 4 different VHH antibody units, i.e., VHH antibody units directed against different Hlg polypeptides, directed against different epitopes of a Hlg polypeptide and/or directed against different *S. aureus* toxins or virulence factors, e.g., *S. aureus* Hla. In certain embodiments, the at least one VHH antibody unit comprises an anti-Hlg VHH antibody as described herein.

In particular embodiments, the hetero-multimeric VHH antibody is a tandem fusion of VHH antibody units comprising a first anti-Hlg VHH antibody unit, particularly an anti-Hlg VHH antibody unit as described herein and a second VHH antibody wherein the first unit is covalently fused to the second unit.

The VHH antibody units are covalently fused to each other either directly or via a linker. In certain embodiments, the linker has a length of 5-50 amino acids, particularly a length of 10-30 amino acids. In certain embodiments, the linker is a flexible linker which may substantially consist or consist of amino acids selected from G, E, S and T.

In certain embodiments, the hetero-multimeric VHH antibody comprises at least two VHH antibody units directed against different Hlg polypeptides selected from HlgA, HlgB and HlgC, e.g., at least one VHH antibody unit against HlgA and at least one VHH antibody unit against HlgB, at least one VHH antibody unit against HlgB and at least one VHH antibody unit against HlgC, or at least one VHH antibody unit against HlgA, at least one VHH antibody unit against HlgB and at least one VHH antibody unit against HlgC.

In certain embodiments, the hetero-multimeric VHH antibody comprises at least two VHH antibody units directed against different epitopes on a Hlg polypeptide, e.g., at least one VHH antibody unit against epitope 1 of HlgB and at least one VHH antibody unit against epitope 2 of HlgB, or at least one VHH antibody unit against epitope 1 of HlgC and at least one VHH antibody unit against epitope 2 of HlgC.

In further embodiments, the hetero-multimeric VHH antibody comprises at least two VHH antibody units directed against different toxins or virulence factors. of *S*. *aureus,* e.g., pore-forming toxins of *S. aureus.* For example, the hetero-multimeric VHH antibody may comprise at least one VHH antibody unite against a Hlg polypeptide as described herein, e.g., a HlgB polypeptide, and at least one VHH antibody unit against another pore-forming toxin. In certain embodiments, the hetero-multimeric VHH antibody comprises at least one VHH antibody unit against *S. aureus* alpha hemolysin (Hla), particularly a VHH antibody unit described in PCT/EP2024/052663, or at least one VHH antibody unit against Leukocidin S (LukS), Leukocidin F (LukF), Leukocidin E (LukE) or Leukocidin D (LukD).

For example, the hetero-multimeric VHH antibody comprises two VHH antibody units which are:
(i) the anti-HlgB VHH antibody Bm29C02 having a VHH sequence as shown in SEQ. ID NO: 5 or a VHH antibody, which is a variant thereof, and
(ii) the anti-Hla VHH antibody Ma7A07 having a VHH sequence as shown in SEQ. ID NO: 93 or a VHH antibody, which is a variant thereof.

In a specific embodiment, a hetero-multimeric VHH antibody is provided as shown in SEQ ID NO: 197.

In a further embodiment, a hetero-multimeric VHH antibody may comprise two VHH antibody units which are:
(i) the anti-HlgB VHH antibody Bm29B04 having a VHH sequence as shown in SEQ. ID NO: 9 or a VHH antibody, which is a variant thereof, and
(ii) the anti-Hla VHH antibody Ma31A12 having a VHH sequence as shown in SEQ. ID NO: 169 or a VHH antibody, which is a variant thereof.

In a further embodiment, a hetero-multimeric VHH antibody may comprise two VHH antibody units which are:
(i) a hetero-multimeric VHH antibody comprising the anti-HlgB VHH antibody Bm29B04 having a VHH sequence as shown in SEQ. ID NO: 9 or a VHH antibody, which is a variant thereof, and the anti-Hla VHH antibody Ma8E03 having a VHH sequence as shown in SEQ. ID NO: 133 or a VHH antibody, which is a variant thereof.

In certain embodiments, the VHH antibody including the hetero-multimeric VHH antibody may be covalently or non-covalently conjugated to a heterologous moiety, which is selected from a labeling group, a capture group, or an effector group, and wherein the heterologous moiety is particularly selected from a fluorescence group, biotin, an enzyme such as a peroxidase, phosphatase, or luciferase, a hapten, an affinity tag, or a nucleic acid such as an oligonucleotide.

In further embodiments, the heterologous moiety is selected from human serum albumin, an albumin-binding moiety, or an Fc fragment of an immunoglobulin molecule, e.g., IgA, IgD, IgE, IgG, IgM, or a subtype thereof. In still further embodiments, the heterologous moiety is selected from one or several non-peptidic polymer moieties, preferably hydrophilic polymer moieties, such as polyethylene glycol (PEG).

In specific embodiments, a hetero-multimeric VHH antibody is provided which further comprises a heterologous polypeptide, e.g., an immunoglobulin Fc fragment and wherein the heterologous polypeptide may be at the C-terminus of the hetero-multimeric VHH antibody.

### Production of VHH antibodies

VHH antibodies including monomeric and multimeric VHH antibodies may be produced as described in WO 2022/023483 and WO 2022/023484, the contents of which are herein incorporated by reference, or by other methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a multimeric VHH antibody.

In a particular embodiment, the VHH antibody is recombinantly produced in a bacterium, e.g., *E. coli* or *Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in *Pichia pastoris, Saccharomyces cerevisiae, Schizosaccharomyces pombe,* or *Hansenula polymorpha,* or in an animal cell, particularly in an insect cell, e.g., an Sf-9 cell, or a mammalian cell, e.g., human or a hamster cell such as HEK293F, CHO or COS-7. For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

### Therapeutic applications

Still a further aspect of the present invention is the use of a VHH antibody as described above in medicine, particularly for therapeutic and/or in vitro or vivo diagnostic use. In certain embodiments, the VHH antibody is used in human medicine.

The VHH antibody of the present invention is useful in the prevention, treatment and/or mitigation of a condition caused by, associated with and/or accompanied by an infection with an Hlg-positive and optionally Hla-positive e *S. aureus.* In certain embodiments, the *S. aureus* is a drug-resistant *S. aureus.*

Exemplary conditions include, but are not limited to a skin infection, pneumonia, bacteremia, sepsis, meningitis, osteomyelitis, and/or endocarditis.

In therapeutic applications, the VHH antibody is administered in an effective amount to a subject in need thereof, particularly to a human subject. The dose will depend on the specific type of agent, e.g., monovalent VHH antibody or multimeric VHH antibody, the type of disease, and the route of administration.

Typically, the VHH antibody is administered as a pharmaceutical composition comprising the active agent and a pharmaceutically acceptable carrier or excipient. Examples of suitable carriers and excipients for formulating antibodies or antibody fragments are well-known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular, or intravenous injection or by infusion. In further embodiments, the pharmaceutical composition is administered locally, e.g., topically, orally, nasally, for example by spraying or intrapulmonary, for example by inhalation as an aerosol.

In certain embodiments, treatment of a patient infected by Hlg-positive and optionally Hla-positive *S. aureus* involves a systemic application of one or several complementary neutralizing anti-Hlg VHH antibodies including hetero-multimeric VHH antibodies, for example, by route of injection. In the case of pneumonia, inhalation might be a preferred application route. In the case of colonization of the nose, application of a spray is possible. In all cases, the treatment should reduce tissue damage by Hlg and the virulence of the pathogen to allow the immune system to clear the infection.

Prophylactic use of anti-Hlg VHH antibodies including hetero-multimeric anti-Hlg/Hla VHH antibodies is considered for a subject that is colonized by *S. aureus* or has been exposed or is likely exposed to the pathogen and/or a subject that is immune-compromised, prepared for surgery and/or has suffered injuries that make an infection likely.

Anti-Hlg VHH antibodies including hetero-multimeric anti-Hlg/Hla VHH antibodies will be effective also against highly antibiotic-resistant strains, and in an advanced state of infection, e.g., in an advanced state of sepsis, where the toxin's action can no longer be cleared by antibiotic treatment alone.

In certain embodiments, a single tandem fusion VHH is administered. In further embodiments, a combination (set) of VHH fusions directed against different epitope classes is administered In further embodiments, a combination (set) of VHH fusions directed against different pore-forming toxins is administered. In further embodiments, a combination (set) of VHH fusions directed against different virulence factors of S. *aureus* is administered.

The VHH antibody may be administered alone as a monotherapy or together, e.g., sequentially, or simultaneously, with a further active agent as a combination therapy, particularly with a further agent that is useful in the prevention, treatment, and/or mitigation of a disorder caused by and/or associated with an infection with S. *aureus* and/or optionally a different bacterium. In certain embodiments, the VHH antibody is administered in combination with an antibiotic against S. *aureus,* e.g., vancomycin, and optionally with a further antibiotic against a different bacterium.

### Diagnostic applications

Diagnostic applications include in vitro methods wherein a VHH antibody or a set of different VHH antibodies is used for detecting Hlg in a sample, e.g., in a body fluid such as saliva, sputum, a lung lavage, a swab, blood, serum or plasma, stool samples or in a tissue or biopsy sample.

Diagnostic applications further include in vivo methods wherein a VHH antibody is used for detecting Hlg in a subject, particularly in a human patient. For diagnostic applications, the VHH antibody may carry a label for direct detection or used in combination with secondary detection reagents, e.g., biotin/streptavidin, detection antibodies, including conventional antibodies or VHH antibodies, for indirect detection according to established techniques in the art.

The present invention encompasses the use of anti-Hlg VHH antibodies including hetero-multimeric VHH antibodies for detecting the toxin with high sensitivity. The availability of different complementary epitope classes can be exploited for highly sensitive detection. In certain embodiments, the detection format comprises the binding of at least 2 different VHH antibodies to an Hlg molecule, particularly the binding of at least 2 different VHH antibodies each directed against a different epitope. In particular embodiments, the detection comprises the use of a set of 2, 3 or 4 different VHH antibodies as described above.

In particular embodiments, the detection comprises a sandwich assay, e.g., a sandwich ELISA. In such a test format, VHH antibodies directed against several complementary epitope classes can be exploited for highly sensitive detection. More specifically, a first VHH antibody may be immobilized to a solid phase for capturing Hlg from the sample, and a second VHH antibody is employed in labelled form for the actual detection. A third and/or fourth VHH antibody can also be used for a prior enrichment step, e.g., by a capture and proteolytic release-approach (Frey & Görlich, 2014b; Frey & Görlich, 2014a; Vera Rodriguez et al., 2019). This allows a rapid and sensitive detection of Hlg which is highly important in life-threatening conditions.

Further, the present invention is explained in more detail by the following Figures and Examples.

### Figure Legends

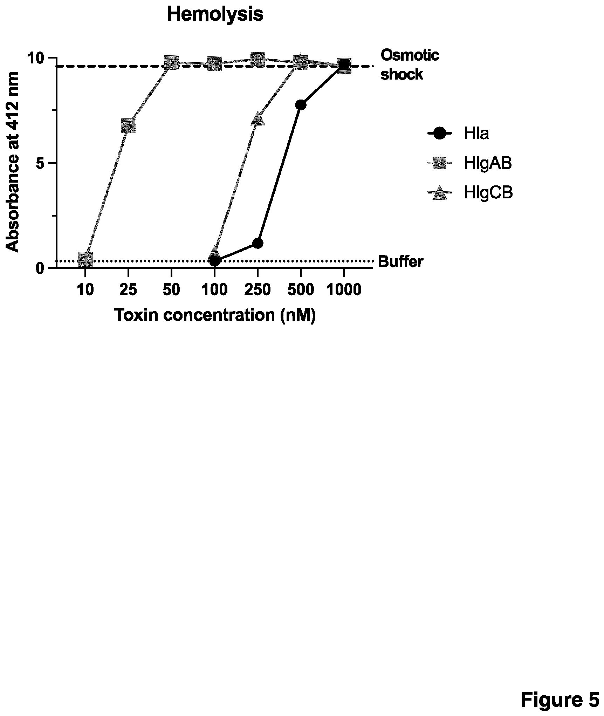

**Figure 1****: Sequence alignment and highlighting of variable regions**
   The figure shows an alignment of sequences from selected VHH antibodies listed in Table 2. Residues that deviate from the consensus are highlighted by a gray background. The three variable CDR regions are indicated.
**Figure 2****: Affinities of anti-HlgB VHHs for HlgB as measured by Bio-layer interferometry (BLI)**
   BLI experiments were performed for HlgB with the indicated VHH antibodies. Curves (grey) were fitted with a mass transport model (black). Calculated dissociation constants (K_{D}s) are shown above the curves.
**Figure 3****: Affinities of anti-HlgA VHHs for HlgA**
   HlgA affinities for the binding of indicated VHHs were measured by BLI.
**Figure 4****: Affinities of anti-HIgC VHHs for HIgC**
   HlgC affinities of indicated VHHs were measured by BLI.
**Figure 5****: Dose dependence of toxin-induced hemolysis**
   Human erythrocytes were dispersed in 96 well plates and incubated with buffer, water (for a hypoosmotic shock) or with indicated amount of toxins for 150 min at 37°C followed by 30 min at 4°C. The plate was centrifuged to pellet intact cells. The supernatants were analyzed on a plate reader for absorbance at 412 nm. Lysed cells release their cytoplasmic contents including heme-bound hemoglobin, which has a maximum absorbance at 412 nm. Dashed lines indicate complete lysis (osmotic shock) and baseline absorbance (buffer).

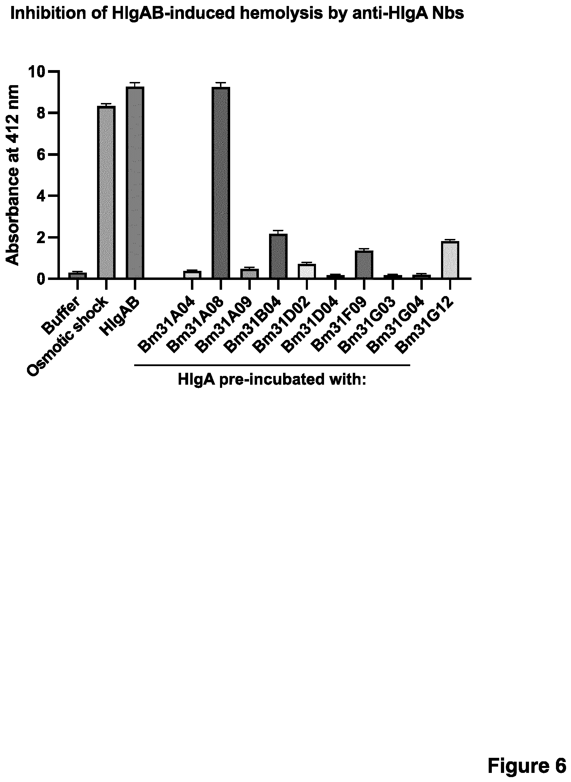

**Figure 6****: Inhibition of HIgAB-induced hemolysis by anti-HigA VHH antibodies.**
   Hemolysis experiment was performed as described in Figure 5 using buffer, water or HlgAB toxin. Where indicated, HlgA was pre-incubated with VHH antibodies before mixing with HlgB.

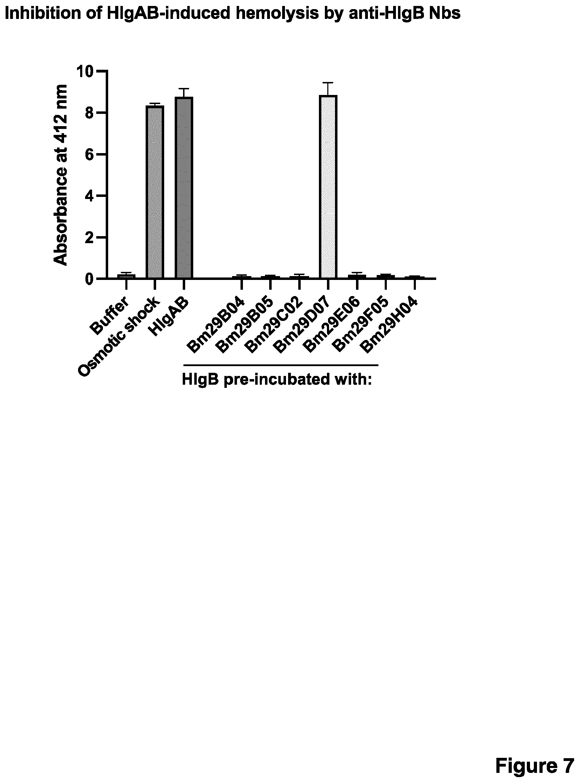

**Figure 7****: Inhibition of HIgAB-induced hemolysis by anti-HlgB VHH antibodies.**
   Hemolysis experiment was performed as described in Figure 5 using buffer, water or HlgAB toxin. Where indicated, HlgB was pre-incubated with VHH antibodies before mixing with HlgA.

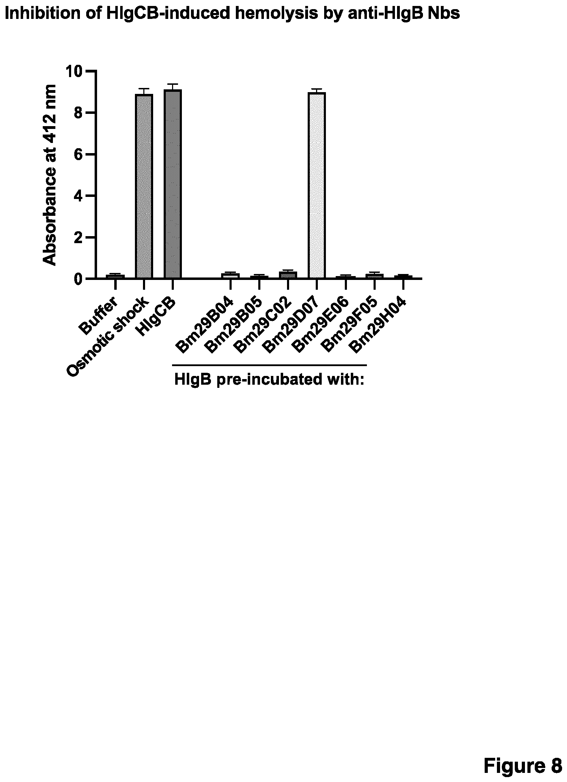

**Figure 8****: Inhibition of HlgCB-induced hemolysis by anti-HlgB VHH antibodies.**
   Hemolysis experiment was performed as described in Figure 5 using buffer, water or HlgCB toxin. Where indicated, HlgB was pre-incubated with VHH antibodies before mixing with HlgC.

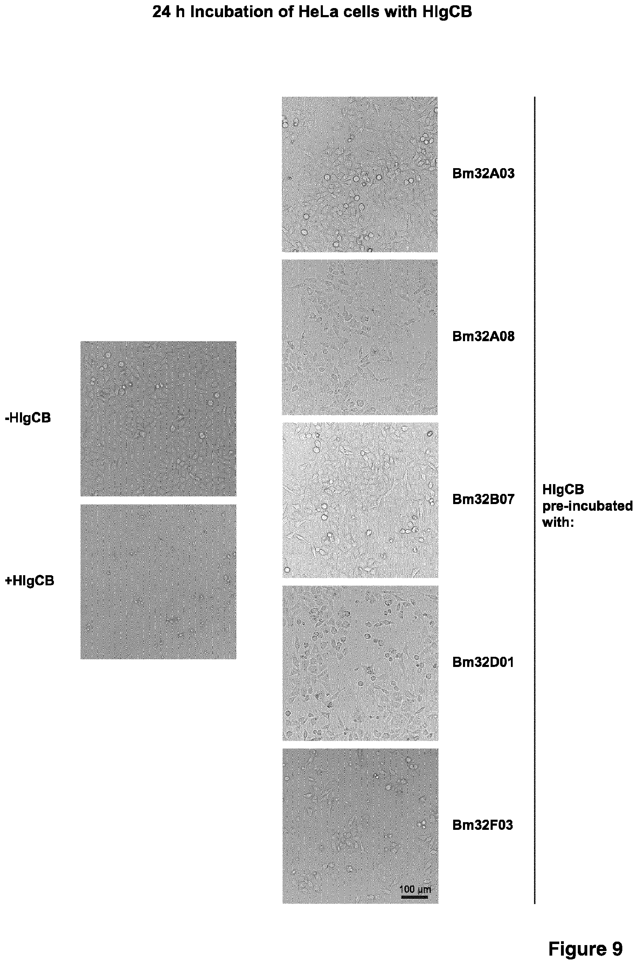

**Figure 9****: Inhibition of HlgCB-induced cell lysis by anti-HlgC VHH antibodies.**
   HeLa cells were dispersed in culture medium into an 8-well ibidi plate. HlgB and HlgC, or HlgB and HlgC pre-incubated with the indicated VHHs were then added as indicated. After overnight incubation at 37 °C and 5% CO₂, the cells were examined by bright field microscopy.

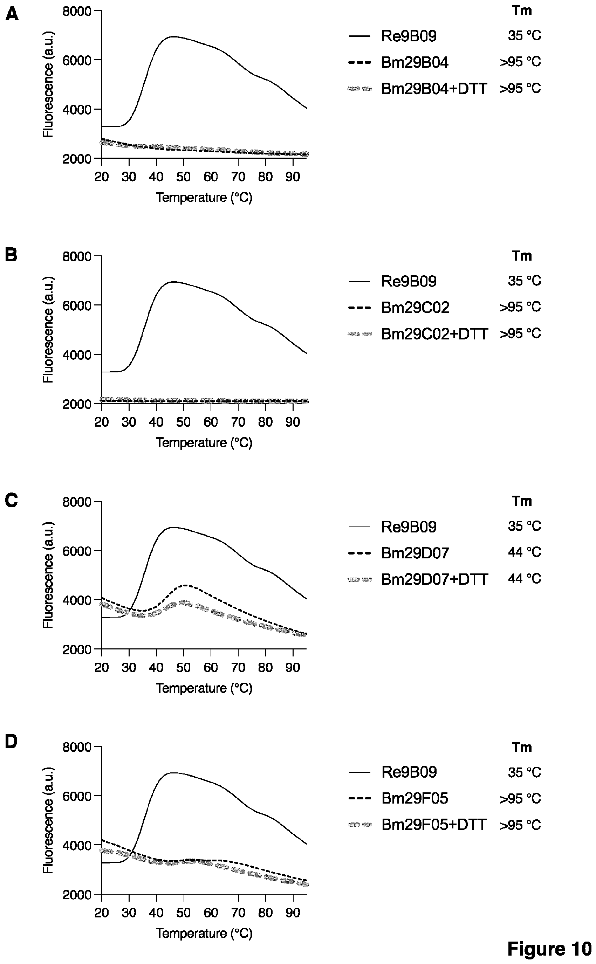

**Figure 10****: Thermal stability of anti-HlgB VHH antibodies.**
   VHH antibodies were subjected to Differential scanning fluorimetry (DSF) as detailed in Example 6. Thermal unfolding is here measured as an enhanced fluorescence of added SYBR Orange following a stepwise increase in temperature with 532 nm excitation and a 555 nm long pass filter. Melting temperatures are defined as the inflection point of the first melting peak.

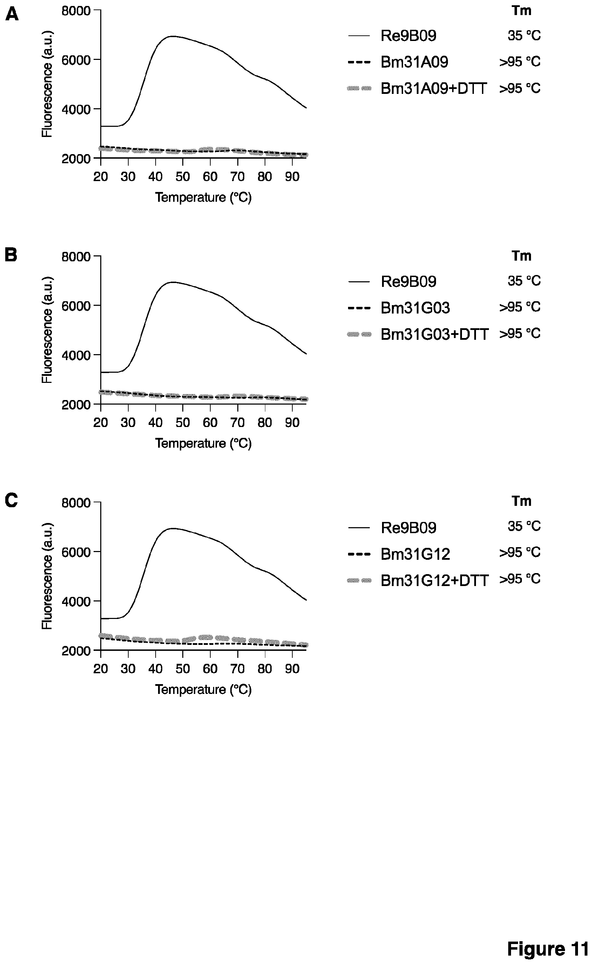

**Figure 11****: Thermal stability of anti-HigA VHH antibodies.**
   Anti-HlgA VHH antibodies were subjected to DSF as detailed in Figure 10 and Example 6.

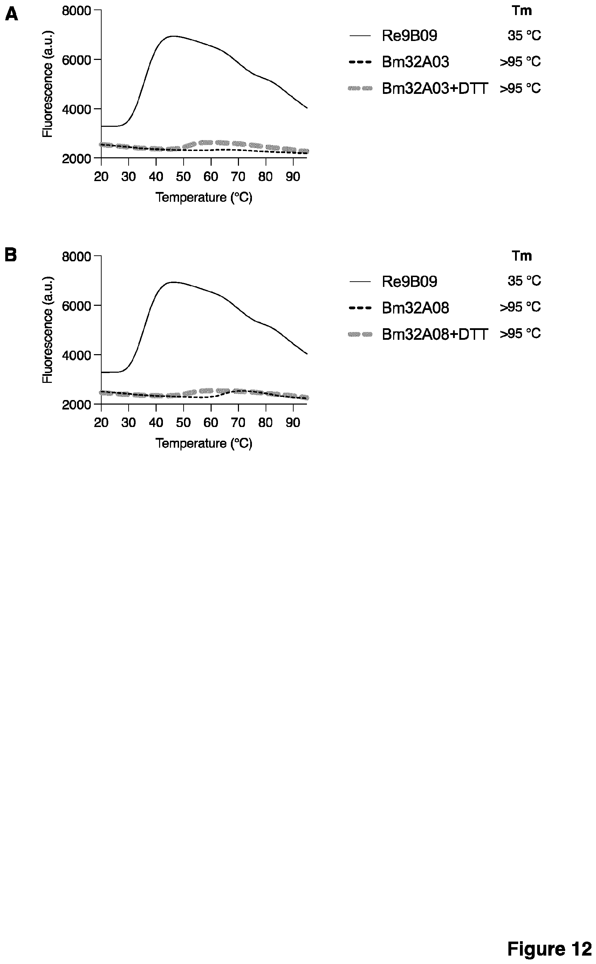

**Figure 12****: Thermal stability of anti-HIgC VHH antibodies.**
   Anti-HlgC VHH antibodies were subjected to DSF as detailed in Figure 10 and Example 6.

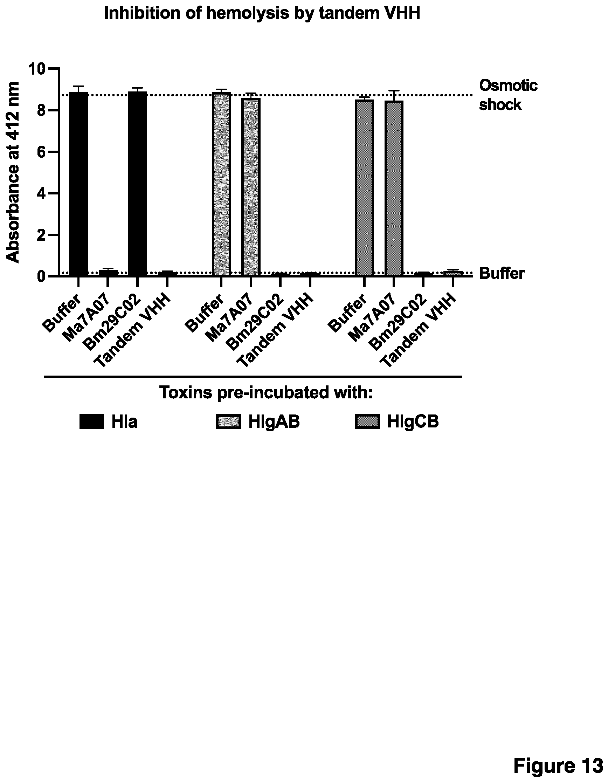

**Figure 13****: Inhibition of hemolysis by tandem VHH.**
   Hemolysis experiment was performed as described in Figure 5 using Hla, HlgAB or HlgCB toxins. Where indicated, toxins were pre-incubated with buffer, anti-Hla VHH Ma7A07, anti-HlgB VHH Bm29C02 or Ma7A07-Bm29C02 tandem fusion. Dashed lines indicate complete lysis (osmotic shock) and baseline absorbance (buffer).

### Sequences of Hla and Hlg components used for assays as described

>Hlg-A (UniProt P0A074 (30-309)) (SEQ ID NO. 1) 
ENKIEDIGQGAEIIKRTQDITSKRLAITQNIQFDFVKDKKYNKDALVVKMQGFISSRTT
YSDLKKYPYIKRMIWPFQYNISLKTKDSNVDLINYLPKNKIDSADVSQKLGYNIGGNF
QSAPSIGGSGSFNYSKTISYNQKNYVTEVESQNSKGVKWGVKANSFVTPNGQVSA
YDQYLFAQDPTGPAARDYFVPDNQLPPLIQSGFNPSFITTLSHERGKGDKSEFEITY
GRNMDATYAYVTRHRLAVDRKHDAFKNRNVTVKYEVNWKTHEVKIKSITPK
>Hlg-B (UniProt P0A077 (36-325)) (SEQ ID NO. 2) 
EGKITPVSVKKVDDKVTLYKTTATADSDKFKISQILTFNFIKDKSYDKDTLVLKATGNI
NSGFVKPNPNDYDFSKLYWGAKYNVSISSQSNDSVNVVDYAPKNQNEEFQVQNTL
GYTFGGDISISNGLSGGLNGNTAFSETINYKQESYRTTLSRNTNYKNVGWGVEAHK
IMNNGWGPYGRDSFHPTYGNELFLAGRQSSAYAGQNFIAQHQMPLLSRSNFNPEF
LSVLSHRQDGAKKSKITVTYQREMDLYQIRWNGFYWAGANYKNFKTRTFKSTYEID
WENHKVKLLDTKETENNK
>Hlg-C (UniProt Q07227 (30-315)) (SEQ ID NO. 3) 
ANDTEDIGKGSDIEIIKRTEDKTSNKWGVTQNIQFDFVKDKKYNKDALILKMQGFISS
RTTYYNYKKTNHVKAMRWPFQYNIGLKTNDKYVSLINYLPKNKIESTNVSQTLGYNI
GGNFQSAPSLGGNGSFNYSKSISYTQQNYVSEVEQQNSKSVLWGVKANSFATES
GQKSAFDSDLFVGYKPHSKDPRDYFVPDSELPPLVQSGFNPSFIATVSHEKGSSDT
SEFEITYGRNMDVTHAIKRSTHYGNSYLDGHRVHNAFVNRNYTVKYEVNWKTHEIK
VKGQN
>Hla (UniProt P09616 (27-319)) (SEQ ID NO. 4)

ADSDINIKTGTTDIGSNTTVKTGDLVTYDKENGMHKKVFYSFIDDKNHNKKLLVIRTK
GTIAGQYRVYSEEGANKSGLAWPSAFKVQLQLPDNEVAQISDYYPRNSIDTKEYMS
TLTYGFNGNVTGDDTGKIGGLIGANVSIGHTLKYVQPDFKTILESPTDKKVGWKVIF
NNMVNQNWGPYDRDSWNPVYGNQLFMKTRNGSMKAADNFLDPNKASSLLSSGF
SPDFATVITMDRKASKQQTNIDVIYERVRDDYQLHWTSTNWKGTNTKDKWTDRSS
ERYKIDWEKEEMTN

### Anti-Hlg VHH antibody sequences

>Bm29C02 (SEQ ID NO. 5) 
QVQLVESGGGLVQPGGSLRLSCTASGFTLDDWAIGWFRQAPGKEREGVSCTSSR
ERSSYYRDSVKGRFTISRDSTSNTVYLQMNELTLEDTAVYYCAADPQPYPCSLGQL
GRYTTWGQGTQVTVSS
>Bm29H04 (SEQ ID NO. 9) 
QVQLVESGGGLVQPGGSLRLSCTASGVSLDDFAIGWFRQAPGKEREGVSCSSSR
DRSEYYRDSVKGRFTISRDSQKNTVDLQMNSLTIEDTAVYYCAADPQPYPCSVGSL
DRYNSWGQGTLVTVSS
>Bm29E06 (SEQ ID NO. 13) 
QVQLVESGGGLVQPGGSLRLSCTASGVSLYDFAVGWFRQAPGKEREGVSCSSSR
DRSEYYRDSVKGRFDIVRDNLKNIIYLQMDNLTIEDTAVYFCAADSQPYPCSLGSLE
RYDSWGQGTQVTVSS
>Bm29F05 (SEQ ID NO. 17) 
QVQLVESGGGLVQPGGSLRLSCTASGVSLYDFAVGWWRQAPGKEREGVSCTSS
RDRSEYYRDSVKGRFTIVRDNLKNTIYLQMDSLTIEDTAVYFCAADSQPYPCSLGSL
ERYDSWGQGIQVTVSS
>Bm29B04 (SEQ ID NO. 21) 
QVQLVESGGGLVQAGGSLRLSCAASGFTFDDYVIGWFRQAPGKEREAVACISNGD
GVANVPNSMKGRFTISSDSAKNTVHLRADSLKADDTGVYYCAAVKERLCVHEGME
YWGKGTQVTVSS
>Bm29B05 (SEQ ID NO. 25) 
QVQLVESGGGLVQAGGSLRLSCAASGFTFDDYVIGWFRQAPGKEREAVACISNGD
GVTNYPNSMRGRFTISSDSAKNTVYLQTDSLKPDDTGVYYCATAKERLCVQGGME
YWGKGTLVTVSS
>Bm29D07 (SEQ ID NO. 29) 
QVQLVESGGGLVQPGGSLRLSCTASSIIVSYYAIGWFRQAPGKGREGVSRITVPAG
NLYYADSVKGRFSITRDDVKNTVYLQMNSFQVEDTATYYCAVSYGDSWVDPSYVD
HWGQGIQVTVSS
>Bm31D04 (SEQ ID NO. 33) 
QVQLVESGGGLVQPGGSLRLSCAASGIIFDFYDMGWYRQAPGKQRELVAGISISGS
TTYADSVKGRFTISRDNAKNTIYLQMNSLKPEDTAVYYCNAHHVDSDYLRGYDYFG
QGTQVTVSS
>Bm31G03 (SEQ ID NO. 37) 
QVQLVESGGGLVQAGGSLRLSCAASGIIFSFYDMGWYRQAPGKQREVVATISISGR
TTYADSVKGRFTISRDDAKNTVYLQMNSLKPEDTAVYICNAQHVDSDYLAGYDYFG
QGTQVTVSS
>Bm31G04 (SEQ ID NO. 41) 
QVQLVESGGGLVQPGGSLRLSCAASGITFTFYDMGWYRQAPGKQRELVATISISSS
TTYADSVKGRFTISRDNAKNTVYLQMNSLKPEDTAVYYCNAQHVDSDYLAGYDYF
GQGTQVTVSS
>Bm31F09 (SEQ ID NO. 45) 
QVQLVESGGALVQPGGSLRLSCAASGFTFDDYVMSWVRQAPGTGLEWVSSIHTA
SSDTYYADSVKDRFTISTDNAKNTLYLQMNSLKPEDTAVYYCAADKAPLTIATMTRD
EYDYWGQGTQVTVSS
>Bm31G12 (SEQ ID NO. 49) 
QVQLVESGGGLVQPGGSLRLSCAAAGFTVDDYVMSWVRQVPGKGREWVSSIHSY
SSDTYYADSVKGRFTISTDNAKNTLYLQMNSLKPEDTAVYYCAADKDPLTIATMTRY
EYDYWGQGTQVTVSS
>Bm31A09 (SEQ ID NO. 53) 
QVQLVQSGGGLVQAGGSLRLSCAASGSTGSIYVMGWFRQAPGKQRELVASITRG
EGSANYADSVKGRFTISRDNAKNTLYLQMNSLKPEDTAVYYCHADTGLGDYSDYA
PHGYKYDYWGQGTQVTVSS
>Bm31D02 SEQ ID NO. 57) 
QVQLVESGGGLVQAGGSLRLSCAASGSTGSIYVMGWFRQAPGKQRELVASITRGE
GSANYADSVKGRFTISRDNAKNTLYLQMNSLKPEDTAVYYCHADTGLGDYSDYAP
HGYKYDYWGQGTQVTVSS
>Bm31A04 (SEQ ID NO. 61) 
QVQLVQSGGGLVQPGGSLRLSCAASESISNIYVMGWFRQAPGKQRELVAAITSSN
NTNYADSVKGRFTISRDNAKNTVYLQMNSLKPEDTAVYYCNADWGLGGYSDYGEY
DYWGQGTQVTVSS
>Bm31A08 (SEQ ID NO. 65) 
QVQLVESGGGLAQPGGSLTLSCAASGVVWSKYVMSWVRQVPEKGFEWVSGIKSD
GSDTYYADSVKGRFTVSGDLAKKTVYLQLNSLKPEDTALYYCVLEYGTSWPPRVE
EYDYWGQGTQVTVSS
>Bm31B04 (SEQ ID NO. 69) 
QVQLVESGGGLVQPGGSLRLSCAASGSSFSIYGMSWYRQAPGNQRELVALINVGD
WTTYADSVKGRFTISRDSAKNTVYLQMNSLKPEDTAVYYCKADLSVSGSSFIRTYW
GQGTQVTVSS
>Bm32A03 (SEQ ID NO. 73) 
QVQLVESGGALVQTGGSLRLSCVTSGSISSIYAMPWFRQAPGKEREFVAAISWHT
NLAYYADSVKGRFTISKDNAKNTVYLQMDSLKPEDTATYYCNVPSKEEYDRTSYYP
TGGSWGQGTQVTVSS
>Bm32B07 (SEQ ID NO. 77) 
QVQLVESGGGLVQTGGSLRLSCATSGRISSIYAMPWFRQAPGKEREFVAAISWHT
DLAYYADSVKGRFTISKDNAKNTVYLQMNSLKPEDTAVYYCNIPSKEEYDRNYYYP
TGGSWGQGTQVTVSS
>Bm32D01(SEQ ID NO. 81) 
QVQLVESGGGLVQTGGSLRLSCATSGRISSIYAMPWFRQAPGKEREFVAAISWHT
DLTYYADSVKDRFTISKDNAKATVYLQMNNLQPEDTAVYYCNVPSKEQYDRTSYYP
TGGSWGQGTQVTVSS
>Bm32A08 (SEQ ID NO. 85) 
QVQLVESGGGLVQAGGSLRVSCAASGDWFIINSMAWFRQAPGKQREQVATISHG
GIPRYADSVKGRFTISRDNSKGAVYLQMNNLKSDDTAMYVCAAGGLDYGFPDPTP
EEFDYWGRGTQVTVSS
>Bm32F03 (SEQ ID NO.89)

QVQLVESGGGLVQAGGSLRVSCAASGDWFIINSMAWFRQAPGKQREQVATISHG
GIPRYADSVKGRFTISRDDSKDAVYLQMNNLKSDDTAMYVCAAGGLDYGFPDPTP
EEFDYWGRGTQVTVSS

### Anti-Hla VHH antibody sequences

>Ma7A07 (SEQ ID NO: 93) 
QVQLVESGGGLVQPGGSLRLSCAASGFTLGDYTLGWFRQAPGKQREGVSSIASS
ALSIYIADSVKGRFTISRDDAKNTIYLHMNNLKPEDTGVYYCARGVDSWRWVPSAM
DLWGKGTLVTVSS
>Ma7A08 SEQ ID NO: 97) 
QVQLVESGGGLVQPGGSLTLSCAASGFTLGDYTLGWFRQAPGKQREGVSSIASSV
LSVYIADSVKGRFTISRDDAKNTIYLHMNNLKPEDTGVYYCARGVDSWRWVPSAM
DLWGKGTLVTVSS
>Ma7B03 (SEQ ID NO: 101 
QVQLVESGGGLVQPGGSLRLSCAASGFTLSNYYVGWFRQAPGKQREGVSSIGSS
DLSIYIADSVKGRFTISRDNAKNTIYLHMNNLKPDDTGVYYCARGTDSWRWVPSAM
DLWGKGTQVTVSS
>Ma7B12 (SEQ ID NO: 105) 
QVQLVESGGGLVQPGGSLRLSCAASGFTLGDYTLGWFRQAPGKQREGVSSIASS
ALSIYIADSVKGRFTISRDNAKNTVYLHMNNLKPEDTGVYYCARGIDNWRWVPSAM
DLWGKGTQVTVSS
>Ma7B01 (SEQ ID NO: 109) 
QVQLVESGGGLVQAGDSLSLSCAASGRTFSNYAMGWFRQAPGKERHFVAVINQIG
DSTWYPDFAKGRFTISRDNAKNTLYLQMNSLKPEDTAVYYCAATDPRSYWRIWPH
EYGYWGQGTQVTVSS
>Ma7C02 (SEQ ID NO: 113) 
QVQLVESGGGLVQAGGSLRLSCAASGRTFSDYAMGWFRQAAGKDRDFVAVITRS
GDSTYYPDSTKGRFTISRDNAKNTMYLQMNSLKPEDTARYYCAATDPTNYWRIWE
HEFDYWGQGTQVTVSS
>Ma7E01 (SEQ ID NO: 117 
QVQLVESGGGSVQAGDSLSLSCVASERTFSNYAMGWFRQAPGKERHFVAVINPIG
EETWYPDFAKGRFTISRDNAKNTLYLQMNSLKPEDTAVYYCAATDPRSYWRIWEH
EFDYWGQGTQVTVSS
>Ma7F02 (SEQ ID NO: 121) 
QVQLVESGGGLVQAGGSLRLSCTVSGSIDSLHTMGWFRQAPGKKRDFVAVVSWS
GGNTYYPDYAKGRFTISRDNTKNTVYLQMNSLKPEDTAIYYCAAESGSGNYWKIW
EHEFDSWGQGTQVTVSS
>Ma7D06 (SEQ ID NO: 125 
QVQLVESGGGLVQAGGSLRLSCAASGSISGIDTMAWFRQIPGKDREFVAVIKWAA
GSTWYPDFVKGRFTVSRDNAKNMVYLQMDSVKPEDTAIYYCAAAAGEKYYYRLFP
YEYDYWGQGTQVTVSS
>Ma7C11 (SEQ ID NO: 129) 
QVQLVESGGGLVRAGGSLRLSCAASGSISSVNGMGWFRQAPGKERLFVAQVSLL
DSSTYYADSVKGRFTISRDDAKNTMYLQMNNVRPEETAVYYCAATKLRIGTAFSSE
YDYWGQGTQVTVSS
>Ma8E03 (SEQ ID NO: 133) 
QVQLVESGGGLVHPGGSLRLSCAASGSSLDHYVIGWFRQVPGKEREGVSCISRSG
GSTNYADSVKGRFTVSRDNVKNMVYLQMNSLEPEDSAEYYCAAQRNLGNFCVLG
WLEYDDWGQGTQVTVSS
>Ma8G02 (SEQ ID NO: 137) 
QVQLVESGGGLVLPGGSLRLSCAASGSSLDHYVIGWFRQYPGKEREGVSCISRSG
GSTNYADSVKGRFTVSRDNVKNMVYLQMNSLEPEDSAEYYCAAQRNLGNFCVLG
WVEYDDWGQGTQVTVSS
>Ma8H03 (SEQ ID NO: 141) 
QVQLVESGGGLVHPGGSLRLSCAASGSSLDHYVIGWFRQVPGKQREGISCISSGG
STNYADSVKGRFTVSRDNVKSTVYLQMNSLAPDDSAEYYCAAQRNLGNFCVLGW
LEYDDWGQGTQVTVSS
>Ma8A05 (SEQ ID NO: 145) 
QVQLVESGGGLVQPGGSLRLSCAASPASGFTLDLYTIAWFLQAPGKEREGVSAISL
SDEITYYSDAVKGRFTISRDSAKNTVFLQMDRLKPEDTGVYYCAIGAFDFILKEADA
DYWGQGTQVTVSS
>Ma8D05 (SEQ ID NO: 149) 
QVQLVESGGGLVQPGGSLRLSCAASPASGFTLDLYTIAWFLQAPGKEREGVSAISL
SDEITYYSDTVKGRFTISRDSAKNTVFLQMDRLKPEDTGVYYCAIGAFDFILKEADA
DYWGQGTQVTVSS
>Ma8D07 (SEQ ID NO: 153) 
QVQLVESGGGLVQPGGSLRLSCAASESIYKLNAMGWYRQAPGKELELVTTISSGG
STFYTDPVKGRFTISTDNAKNTVYLQMRSLKPEDTAMYYCAANPTYNHYSARGQG
TQVTVSS
>Ma8D08 SEQ ID NO: 157 
QVQLVESGGGFVQAGGSLRLSCAASESIYKLNAMGWYRQAPGKELELVTTISSGG
STFYTDPVKGRFTISTDNAKNTVYLQMRSLKPEDTAMYYCAANPTYNHYSARGQG
TQVTVSS
>Bm28F09 (SEQ ID NO: 161) 
QVQLVESGGGLVEPGGSLTLSCAASGFTSKNYYIGWFRQAPGKYREGVASIGASD
GSLYIADSVKGRFTISSDNAKNTVYLHPRNLKPEDSGVYYCATGRDSWRWVPSAM
DYWGKGIQVTVSS
>Bm28H01 (SEQ ID NO: 165 
QVQLVESGGGLVQPGGSLTLSCAASGFTLGDRTLGWFRQVAGKQREGVASIASG
VLSAYIADSVKGRFTISRDDAKNTIYLHMNNLKPDDTGVYYCARGVDSWRWVPST
MDLWGKGILVTVSS
>Ma31A12 (SEQ ID NO: 169) 
QVQLVESGGGLVQPGGSLRLSCAASAASGFTLDLYTIAWFLQAPGKEREGVSAISL
SDEITYYSDAVKGRFTISRDSAKNTVFLQMDRLKPEDTGVYYCAIGAFDFILKEVDA
DYWGQGTQVTVSS
>Ma31B06 (SEQ ID NO: 173) 
QVQLVQSGGGLVQPGGTLRLSCAASPASGFTLDLYTIAWFLQAPGKEREGVSAISL
SDEITYYSDAVKGRFTISRDSAKNTVFLQMDRLKPEDTGVYYCAIGAFDFILKEADA
DYWGQGTQVTVSS
>Bm28C04 (SEQ ID NO: 177) 
QVQLVESGGGSVQPGNSLRLSCKVSGRTFNSYALGWFRRRPGDQRDFVASIIRST
GGTSYADSVKGRFTISYDNVQNTVDLQMNSLEPEDTAIYYCAGGDGHIYAFGYDY
WGQGTQVTVSS
>Bm28H07 (SEQ ID NO: 181) 
QVQLVESGGGSVQPGNSLRLSCKVSGRTFNSYALGWFRRRPGDQRDFVASIIRST
GGTSYADSVKGRFTISYDNVQNTVDLQMSSLEPEDTAIYYCAGGDGHIYAFGYDY
WGQGTQVTVSS
>Bm28C09 (SEQ ID NO:185) 
QVQLVESGGSVVQAGGSLRLSCTASGRNFNSYAMGWFRRPPGEQRDFIAAIIRST
GKTTYADSDSVKGRFTISKDSSGNTVYLQMNNLKPEDTAIYYCAGGDAGPYAFGY
DYWGQGTQVTVSS
>Bm28H12 (SEQ ID NO: 189) 
QVQLVESGGSVVQAGGSLRLSCTASGRNFNSYAMGWFRRPPGEQRDFIAAIIRST
GKTTYADSDSVKGRFTISKDSSGNTVYLQMNNLKPEDTAIYYCAGGDAGPYAFGY
DYWGQGTQVTVSS
>Bm28A12 (SEQ ID NO: 193)

QVQLVESGGGLVQAGGSLRLSCVVSGRALSSYNLAWFRQSPGKEREWIATIMPSA
DKAHYPDFLEGRFTISGDNTENTLYLQMNSLKPEDTAIYYCAAREPSYYHALFEYEY
NLWGQGTQVTVSS

### Tandem fusions

>Ma7A07-Bm29C02-Fc fusion (SEQ ID NO. 197)

ETGGSQVQLVESGGGLVQPGGSLRLSCAASGFTLGDYTLGWFRQAPGKQREGVS
SIASSALSIYIADSVKGRFTISRDDAKNTIYLHMNNLKPEDTGVYYCARGVDSWRW
PSAMDLWGKGTLVTVSSTSTSGEGEGGGEGGSQVQLVESGGGLVQPGGSLRLS
CTASGFTLDDWAIGWFRQAPGKEREGVSCTSSRERSSYYRDSVKGRFTISRDSTS
NTVYLQMNELTLEDTAVYYCAADPQPYPCSLGQLGRYTTWGQGTQVTVSSGTENL
YFQGKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKV
SNKALPAPIEKTISKAKGQPREPQVYTLPPSRDELTKNQVSLTCLVKGFYPSDIAVE
WESNGQPENNYKATPPVLDSDGSFFLYSKLTVDKSRWQQGNVFSCSVMHEALHN
HYTQKSLSLSPGKSGTSHHHHHH

### Examples

The primary aim was to obtain VHH antibodies that inhibit Gamma-Hemolysin (Hlg) activity. To this end, the inventors recombinantly produced Hlg components and immunized one alpaca with the B component (HlgB) and another alpaca with the A and C components (HlgA and HlgC) four times at three-weeks intervals. Please note that toxicity requires AB or BC components together, so the immunogens used were not toxic for the animal.

One week after the final immunization, blood samples were taken, lymphocytes were isolated, and mRNAs were purified and reverse transcribed. VHH-coding regions were amplified by nested PCR and cloned as cDNAs into an M13 phagemid, yielding libraries with >100 million independent clones.

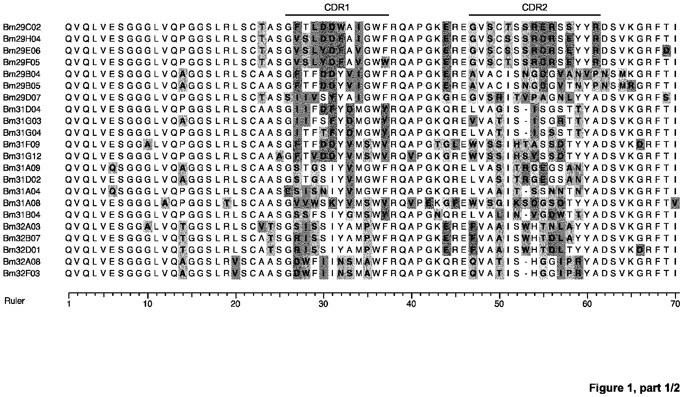

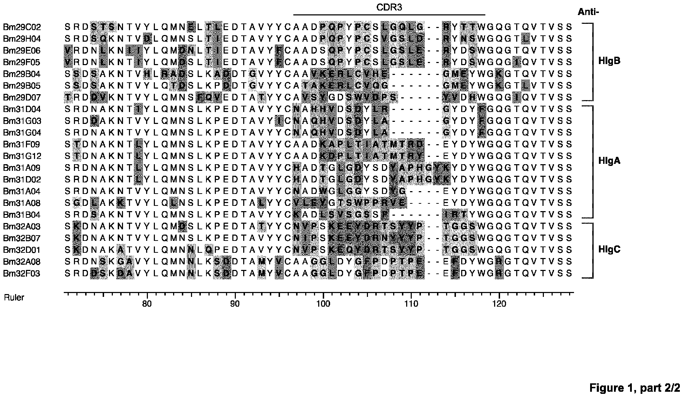

In the next steps, two rounds of phage display were performed using the individual components as baits. Selected VHH antibodies were classified by sequence similarity, cloned into *E*. *coli* expression vectors, produced by periplasmic expression, and purified (the Bm29 series targets HlgB, the Bm31 series targets HlgA, and the Bm32 series targets HlgC; see Figure 1 for specific sequences).

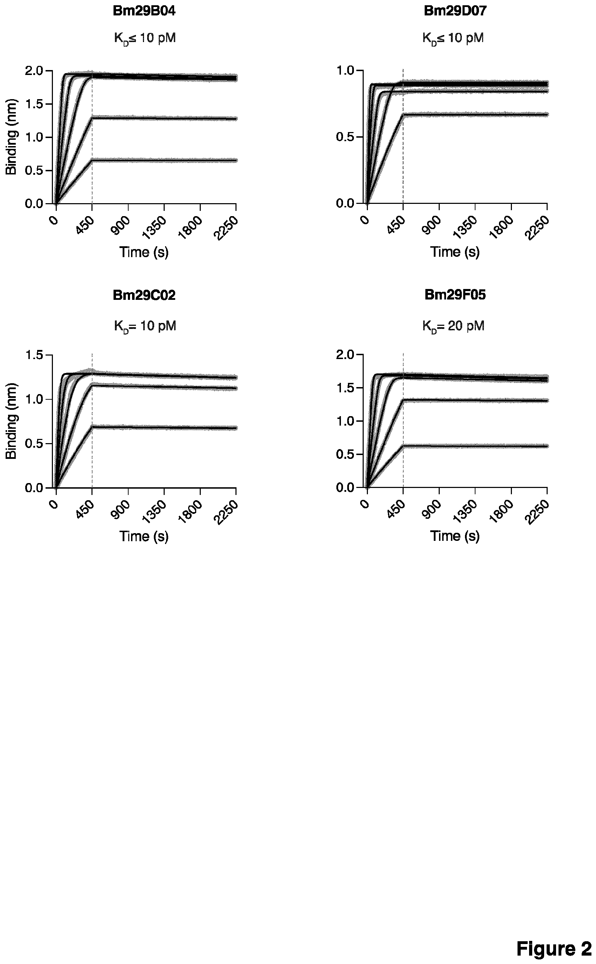

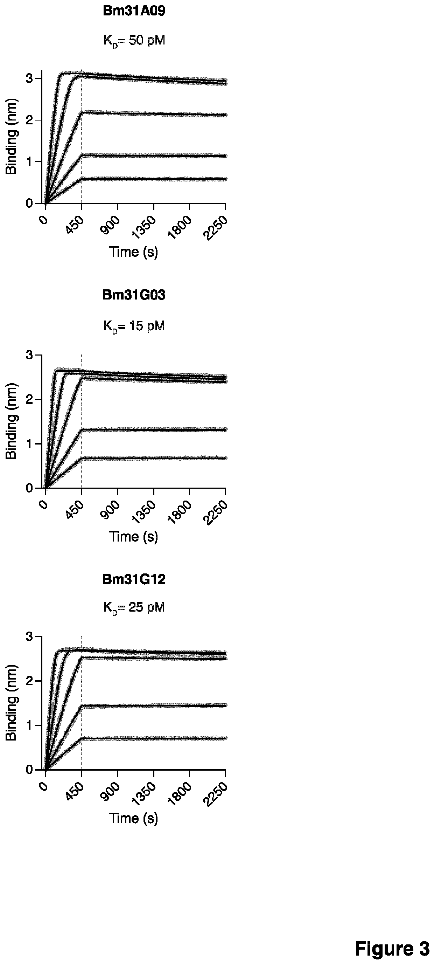

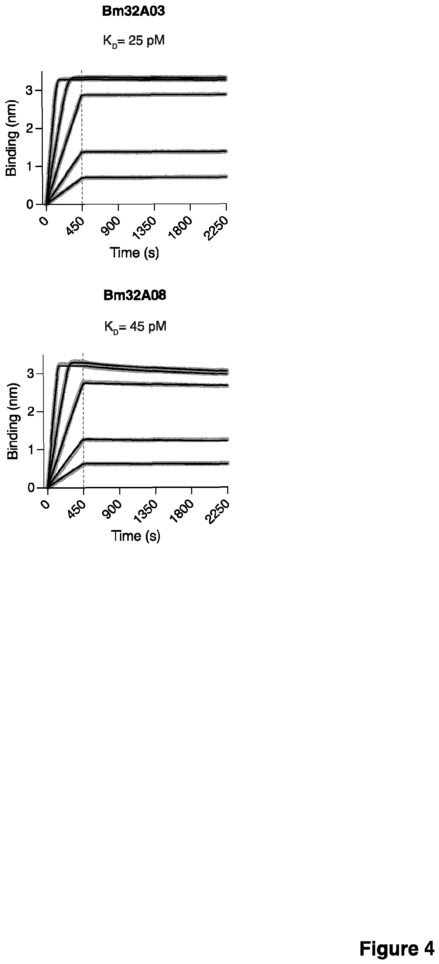

The inventors then analyzed interactions between the VHHs and Hlg components by Bio-Layer Interferometry (BLI; (Abdiche et al., 2008)). Results are shown in Figures 2-4, as well as in Table 1. These measurements revealed extremely high affinities (up to 10 pM, please note that the BLI setup does not allow to discern affinities below 10 pM) with high on-rates (mostly around 10⁵ - 10⁶ M⁻¹·s⁻¹), and non-detectable or nearly non-detectable dissociation rates for most of the VHHs.

To assess if and how many complementary binding sites these VHHs have on the respective Hlg components, the inventors performed binning experiments using BLI. In these experiments, one Hlg component was allowed to bind to immobilized VHH, and then the binding of another VHH was monitored. 'Binning' identified three complementary epitopes (epitopes 1-3) for HlgB, and two complementary epitopes for HlgA and HlgC, where epitope 1-binding VHH antibodies could bind simultaneously with either epitope 2-binders or epitope 3-binders. Likewise, epitope 2- and 3-binders could also bind simultaneously.

In the following experiments, it was tested whether these VHH antibodies could neutralize Hlg. Red blood cells (RBCs or erythrocytes) are susceptible to HIgAB's lytic activity. Moreover, the high concentration of Heme (as part of oxygen-carrying hemoglobin) in RBCs makes the detection of cell lysis very straightforward. Therefore, the inventors performed HlgAB-induced hemolysis experiments using human RBCs. They observed that 50 nM HlgAB led to complete cell lysis (Figure 5).

HlgCB does not primarily target human erythrocytes as these cells lack an HlgC receptor. Nevertheless, *in vitro,* high concentrations of HlgCB shows hemolytic activity probably through weak binding to the HlgA receptor DARC (Spaan et al., 2015a). In fact, the inventors found that 500 nM HlgCB was sufficient for complete hemolysis (Figure 5).

When 150 nM anti-HIgA VHHs were pre-incubated with 50 nM HIgA, all epitope 1-binders impeded HlgAB-induced toxicity. Bm31A04, Bm31A09, Bm31D04, Bm31G03 and Bm31G04 protected cells from hemolysis completely (Figure 6 and Table 1).

All epitope 1- and 2-binders of anti-HIgB VHHs also blocked HlgAB-induced hemolysis completely when pre-incubated with HlgB. Bm29D07, the only epitope 3-binder, failed to protect cells (Figure 7 and Table 1).The inventors also tested these anti-HIgB VHHs in the HlgCB-induced hemolysis experiments and found that all epitope 1- and 2-binders potently inhibited cell lysis (Figure 8). However, when anti-HlgC VHHs were tested for hemolysis inhibition, all VHHs failed to protect cells from hemolysis in this assay.

In human hosts, HlgCB targets leukocytes and other C5aR1-expressing cells, rather than RBCs. It was thus well possible that HlgC-C5aR1 and HlgC-DARC interactions had different modes of action and/or receptor recognition mechanisms. Therefore, the inventors tested HlgCB also in a C5aR1 context. HeLa cells were reported to be affected by HlgCB toxin (Thelestam et al., 1973). Indeed, when added to cultured Hela cells, HlgCB caused death and detachment of the cells from the plate surface (Figure 9). Preincubation of the HlgCB toxin with anti-HlgC VHHs prevented cell death. Bm32A03, Bm32A08 and Bm32B07 were particularly effective in neutralizing toxicity and protecting cells. When tested in analogous conditions, epitope 1- and 2-binders amongst the anti-HIgB VHH antibodies also blocked cell death completely. The consistent results in RBCs and HeLa cells might indicate that the neutralizing activity of anti-HIgB VHH antibodies is independent of the target receptor but acts directly on the HlgC interaction.

The anti-Hlg VHH antibodies were also tested for their thermostability by thermal shift assays or specifically by differential scanning fluorimetry (Goldberg et al., 2011). The method exploits that melting (thermal unfolding) of a protein exposes aromatic/hydrophobic residues (from its hydrophobic core), which then bind and enhance the fluorescence of the added SYPRO Orange dye.

Figures 10-12 show such analyses for the disclosed VHH antibodies, with slow heating from 20°C to 95°C. Re9B09 (described in WO2022/023483), which was expressed in the cytoplasm of NEB Express cells and hence lacks disulfide bonds, melts already at 35 °C.

The majority of VHHs (Bm29C02, Bm29H04, Bm29F05, Bm29B04, Bm29B05, Bm31D04, Bm31G03, Bm31G04, Bm31F09, Bm31G12, Bm31A09, Bm31D02, Bm31A04, Bm32A03, Bm32B07, Bm32F03; see Table 1) showed only a negligible unfolding signal and thus no melting (the small fluorescence peak did not or hardly exceed the measured fluorescence at 20°C). The very low melting amplitudes indicate resistance to melting and thus full thermal stability. We observed that most of the VHHs retained their thermostability even in the presence of the disulfide bond-reducing agent DTT (dithiothreitol) (Figure 10-12, and Table 1).

Having generated VHH antibodies against Hla (disclosed in PCT/EP2024/052663) and Hlg toxins, the inventors explored whether a single polypeptide, i.e., tandem fusion of two VHH antibodies, could neutralize multiple toxins. Figures 7 and 8 show that anti-HlgB VHHs can block both HlgAB- and HlgCB-induced hemolysis. The inventors therefore reasoned that a fusion of a single anti-Hla VHH antibody with a single anti-HlgB VHH antibody could block three toxins simultaneously.

Ma7A07 (SEQ ID NO: 93) is a hyperthermostable VHH antibody that binds Hla with a ≤10 pM affinity as described in PCT/EP2024/052663. Bm29C02 (SEQ ID NO: 5) is also hyperthermostable and binds HlgB with 10 pM affinity (Table 1 and Figure 2). Hence, Ma7A07 was fused trough a 15 amino acid linker in tandem with Bm29C02 and a human IgG1 Fc region. The tandem was then produced recombinantly in Expi293F^{™} human cells and tested in hemolysis inhibition assays. While individual VHH antibodies blocked only their cognate toxins, the resulting fusion fully protected RBCs from all toxins, namely Hla, HlgAB, and HlgCB (Figure 13).

### Example 1: Kinetic measurements by Bio-layer interferometry (BLI)

For affinity measurements, VHHs were produced with a C-terminal Avi-Biotin-tag and enzymatically biotinylated by recombinant BirA (Beckett et al., 1999). They were immobilized at a concentration of 100 nM for 120 seconds on High Precision Streptavidin biosensors of an Octet RED96e instrument (ForteBio/Sartorius), using Phosphate-Buffered Saline (PBS) pH 7.4, 0.02% (w/v) Tween 20 and 0.1% (w/v) bovine serum albumin (BSA) as the assay buffer. 50, 25, 12.5, 6.25 or 3.125 nM of the indicated Hlg component were then allowed to bind for 450 seconds, followed by a dissociation step of 1800 seconds. Binding and dissociation were recorded as wavelength shifts (in nm). Baselines were recorded by measuring a 'minus Hlg control' in parallel. On-rates, off-rates, and dissociation constants (K_{D}s) were calculated using the Octet Data Analysis HT 12.0 software by fitting a mass transport model to the data. This revealed very tight binding of the analyzed VHHs to their cognate Hlg components.

The results are summarized in Table 1 and shown in Figure 2-4.

### Example 2: Identification of complementary Hlg epitopes

Epitope binning experiments were performed in a similar setup as described in Example 1. One biotinylated VHH (VHH1) was immobilized on biosensors. The biosensors were dipped into wells containing 100 nM of the corresponding Hlg component for 300 seconds and then incubated for 300 seconds with a second VHHs (VHH2) at 100 nM concentration. When VHH2 showed no binding, VHH1 and VHH2 were considered to belong to the same epitope class. By repeating this for all VHH class representatives, VHH antibodies were classified by their epitope class. Three epitopes were identified for HlgB, and two epitopes for HlgA and HlgC each.

The results are summarized in Table 1.

### Example 3: Erythrocyte hemolysis assays

150 µl of the indicated Hla, HlgAB, and HlgCB dilutions (10 nM to 1 µM) in assay buffer (PBS pH 7.4, 0.4% w/v glucose, 25 mM EDTA) were pipetted in duplicate into a 96-well plate before 50 µL erythrocytes (4×10⁸ cells) were added to each well. The erythrocytes (AB RH+) were obtained from the German Red Cross.

The plate was incubated for 2.5 hours with shaking at 900 rpm at 37°C, followed by another 30 min incubation at 4°C. The plate was then centrifuged for 10 minutes in a swing-out rotor at 400 g and 4°C. 100 µL of the supernatant was transferred to another 96-well plate. Hemoglobin concentrations were measured photometrically at 412 nm (OD412). To determine baseline and maximum absorbance values, cells were incubated with buffer alone or water (for hypo-osmotic lysis).

The results are shown in Figure 5. Note that diluting erythrocytes in water leads to complete lysis, and that 50 nM HlgAB resulted in the same complete cell lysis. On the other hand, 500 nM HlgCB was required for complete lysis. This is consistent with the fact that the affinity of HlgC for DARC is ten times lower than that of HlgA (229 nM vs 29 nM; (Spaan et al., 2015a)). Hla required the highest concentration (1 µM) for complete hemolysis.

### Example 4: Hemolysis inhibition assays

To assess if the disclosed anti-Hlg VHH antibodies neutralize toxin activity, hemolysis inhibition assays were performed using human erythrocytes.

To test for HlgAB neutralization, HlgA or HlgB (66.6 nM each) was pre-incubated with 200 nM of anti-HlgA or anti-HIgB VHH antibodies for 30 min, before addition to erythrocytes. Cells incubated with buffer, water, and HlgAB in the absence of any VHH served as controls. Each condition was assayed in quadruplicate. Erythrocytes were processed, and hemoglobin levels in the supernatant were measured, as described in Example 3. Please note that the concentrations of active toxin and VHH s in the final assay solution were 50 nM and 150 nM, respectively.

For HlgCB neutralization, 666 nM HlgB was pre-incubated with 2 µM anti-HIgB VHH antibodies for 30 min, and then mixed with 666 nM HlgC before the hemolysis assay. The data showed that all epitope 1-binders of HlgA and all epitope 1- and 2-binders of HlgB blocked Hlg-AB-induced toxicity. Remarkably, all Bm29B04, Bm29C02, Bm31D04, and Bm31G03 class members completely blocked hemolysis (Figures 6 and 7, and Table 1). Similarly, Bm29B04 and Bm29C02 class members of anti-HIgB VHH antibodies fully protected cells from HlgCB-induced hemolysis (Figure 8 and Table 1).

### Example 5: HeLa-based cytotoxicity inhibition assays

Erythrocytes are not the primary target of the HlgCB toxin because these cells lack the main receptor, and the affinity of HlgCB for DARC is relatively weak. The toxin rather targets leukocytes and other tissues, including HeLa cells, which express C5aR1. To test whether the anti-HIgB and anti-HlgC VHH antibodies protect cells from the HlgCB toxin, neutralization assays were performed with cultured HeLa cells.

To assess neutralization, 240 µM HlgC was pre-incubated with 1.2 µM of the indicated anti-HlgC VHH antibodies for 30 min. The mixes were supplemented with 240 nM HlgB, before adding 50 µL of them to 250 µL medium with cultured HeLa cells (yielding final toxin and VHH concentrations of 40 nM and 200 nM, respectively). As controls, cells treated with media alone or with HlgCB in the absence of any VHH. Each condition was assayed in duplicate. Cells were incubated overnight at 37°C with 5% CO₂, and then imaged using a (20x objective) brightfield microscope.

Almost all of the toxin-treated cells died and detached from the surface after overnight incubation, whereas untreated cells proliferated normally. The images showed that pre-incubation of the toxin with anti-HlgC VHH antibodies prevented cell death. In particular Bm32A03, Bm32A08, and Bm32B07 were very effective at protecting cells, as corresponding cells were not different from untreated cells (Figure 9).

Anti-HIgB VHH antibodies were also tested under identical conditions. Similar to the results shown in Example 4 and Figure 8, all epitope 1- and 2-binders potently inhibited cell death.

### Example 6 - Thermostability measurement of VHH antibodies

VHH antibodies were subjected to Differential scanning fluorimetry (DSF), which exploits that thermal unfolding exposes aromatic/hydrophobic residues, which then bind and enhance the fluorescence of the added SYPRO Orange dye. Assays were performed in a volume of 20 µl, at 1 mg/ml VHH concentration in 50 mM Tris/HCl, 150 mM NaCl (pH 8.0 at 20°C), and 1x dye (diluted from a 5000x stock; Life Technologies), in the absence or presence of 10 mM DTT (dithiothreitol). Two sample replicates were pipetted into a Hard-Shell^{®} 96-well plate (Bio-Rad). The plate was sealed with transparent MicroSeal^{®} 'B' Seal (Bio-Rad), briefly centrifuged to remove air bubbles, and placed in a CFX96 Real-Time System (C1000 Thermal Cycler, BioRad). The samples were incubated for 5 min at 20°C and then heated in 1°C increments of 45 seconds up to 95°C. Fluorescence was measured at the end of each step with 532 nm excitation and a 555 nm long pass filter. Melting temperatures are defined as the inflection point of the first melting peak.

The results are shown in Table 1 and Figure 10-12. VHH antibody Re9B09, produced in reducing cytosol and hence lacking disulfide bonds, melts already at 35°C. VHH antibodies Bm29D07, Bm29E06, Bm31A08, and Bm31B04 melt similarly at 44°C, 58°C, 67°C and 72°C, respectively, while majority of VHH antibodies showed only a negligible melting amplitude and thus remained stable throughout 95°C. Remarkably, Bm28B04, Bm29C02, Bm31A09, Bm31G03, Bm32A03, and Bm32A08 were resistant to melting even in the presence of disulfide-reducing DTT.

### Example 7: Inhibition of toxin-induced hemolysis by VHH tandem

Anti-Hla VHH Ma7A07, anti-HIgB VHH Bm29C02, and a Ma7A07-Bm29C02-Fc tandem were used in neutralization experiments of the Hla, HlgAB, and HlgCB toxins. 1 µM HLA, 50 nM HlgAB, or 500 nM HlgCB was pre-incubated with a 3-fold excess of VHH antibodies. Otherwise experiments were performed analogously to those described in Examples 3 and 4, and Figures 5-8. Figure 13 documents the results. While Ma7A07 neutralized only Hla and Bm29C02 only Hlgs, the tandem VHH blocked all toxins and fully protected cells from lysis.

### References

Abdiche, Y., Malashock, D., Pinkerton, A., & Pons, J. (2008). Determining kinetics and affinities of protein interactions using a parallel real-time label-free biosensor, the Octet. Anal Biochem, 377(2), 209-217. https://doi.org/10.1016/j.ab.2008.03.035
Beckett, D., Kovaleva, E., & Schatz, P. J. (1999). A minimal peptide substrate in biotin holoenzyme synthetase-catalyzed biotinylation. Protein Sci, 8(4), 921-929. https://doi.org/10.1110/ps.8.4.921
Cassat, J. E., & Skaar, E. P. (2013). Iron in infection and immunity. Cell Host Microbe, 13(5), 509-519. https://doi.org/10.1016/j.chom.2013.04.010
Cheung, G. Y. C., Bae, J. S., & Otto, M. (2021). Pathogenicity and virulence of Staphylococcus aureus. Virulence, 12(1), 547-569. https://doi.org/10.1080/21505594.2021.1878688
Côrtes, M. F., Costa, M. O., Lima, N. C., Souza, R. C., Almeida, L. G., Guedes, L. P. C., Vasconcelos, A. T., Nicolás, M. F., & Figueiredo, A. M. (2017). Complete genome sequence of community-associated methicillin-resistant Staphylococcus aureus (strain USA400-0051), a prototype of the USA400 clone. Mem Inst Oswaldo Cruz, 112(11), 790-792. https://doi.org/10.1590/0074-02760170128
Diep, B. A., Gill, S. R., Chang, R. F., Phan, T. H., Chen, J. H., Davidson, M. G., Lin, F., Lin, J., Carleton, H. A., Mongodin, E. F., Sensabaugh, G. F., & Perdreau-Remington, F. (2006). Complete genome sequence of USA300, an epidemic clone of community-acquired meticillin-resistant Staphylococcus aureus. Lancet, 367(9512), 731-739. https://doi.org)/10.1016/S0140-6736(06)68231-7
Divyakolu, S., Chikkala, R., Ratnakar, K. S., & Sritharan, V. (2019). Hemolysins of Staphylococcus aureus-An Update on Their Biology, Role in Pathogenesis and as Targets for Anti-Virulence Therapy. Advances in Infectious Diseases, 09(02), 80-104. https://doi.org/10.4236/aid.2019.92007
Earls, M. R., Shore, A. C., Brennan, G. I., Simbeck, A., Schneider-Brachert, W., Vremerǎ, T., Dorneanu, O. S., Slickers, P., Ehricht, R., Monecke, S., & Coleman, D. C. (2019). A novel multidrug-resistant PVL-negative CC1-MRSA-IV clone emerging in Ireland and Germany likely originated in South-Eastern Europe. *Infect Genet Evol,* 69, 117-126. https://doi.org/10.1016/j.meegid.2019.01.021
Frisch, M. B., Castillo-Ramirez, S., Petit, R. A., Farley, M. M., Ray, S. M., Albrecht, V. S., Limbago, B. M., Hernandez, J., See, I., Satola, S. W., & Read, T. D. (2018). Invasive Methicillin-Resistant Staphylococcus aureus USA500 Strains from the U.S. Emerging Infections Program Constitute Three Geographically Distinct Lineages. mSphere, 3(3), e00571-17.
Goldberg, D. S., Bishop, S. M., Shah, A. U., & Sathish, H. A. (2011). Formulation development of therapeutic monoclonal antibodies using high-throughput fluorescence and static light scattering techniques: role of conformational and colloidal stability. J Pharm Sci, 100(4), 1306-1315. https://doi.org/10.1002/jps.22371
Güttler, T., Aksu, M., Dickmanns, A., Stegmann, K. M., Gregor, K., Rees, R., Taxer, W., Rymarenko, O., Schünemann, J., Dienemann, C., Gunkel, P., Mussil, B., Krull, J., Teichmann, U., Groß, U., Cordes, V. C., Dobbelstein, M., & Görlich, D. (2021). Neutralization of SARS-CoV-2 by highly potent, hyperthermostable, and mutation-tolerant nanobodies. EMBO J, 40(19), e107985. https://doi.org/10.15252/embj.2021107985
Ippolito, G., Leone, S., Lauria, F. N., Nicastri, E., & Wenzel, R. P. (2010). Methicillin-resistant Staphylococcus aureus: the superbug. International Journal of Infectious Diseases, 14, S7-S11. https://doi.org/10.1016/j.ijid.2010.05.003
Lodise, T. P., Graves, J., Evans, A., Graffunder, E., Helmecke, M., Lomaestro, B. M., & Stellrecht, K. (2008). Relationship between vancomycin MIC and failure among patients with methicillin-resistant Staphylococcus aureus bacteremia treated with vancomycin. Antimicrob Agents Chemother, 52(9), 3315-3320. https://doi.org/10.1128/AAC.00113-08
Côrtes, M. F., Costa, M. O., Lima, N. C., Souza, R. C., Almeida, L. G., Guedes, L. P. C., Vasconcelos, A. T., Nicolás, M. F., & Figueiredo, A. M. (2017). Complete genome sequence of community-associated methicillin-resistant Staphylococcus aureus (strain USA400-0051), a prototype of the USA400 clone. Mem Inst Oswaldo Cruz, 112(11), 790-792. https://doi.org/10.1590/0074-02760170128
Diep, B. A., Gill, S. R., Chang, R. F., Phan, T. H., Chen, J. H., Davidson, M. G., Lin, F., Lin, J., Carleton, H. A., Mongodin, E. F., Sensabaugh, G. F., & Perdreau-Remington, F. (2006). Complete genome sequence of USA300, an epidemic clone of community-acquired meticillin-resistant Staphylococcus aureus. Lancet, 367(9512), 731-739. https://doi.org/10.1016/S0140-6736(06)68231-7
Earls, M. R., Shore, A. C., Brennan, G. I., Simbeck, A., Schneider-Brachert, W., Vremerǎ, T., Dorneanu, O. S., Slickers, P., Ehricht, R., Monecke, S., & Coleman, D. C. (2019). A novel multidrug-resistant PVL-negative CC1-MRSA-IV clone emerging in Ireland and Germany likely originated in South-Eastern Europe. *Infect Genet Evol,* 69, 117-126. https://doi.org/10.1016/j.meegid.2019.01.021
Frisch, M. B., Castillo-Ramirez, S., Petit, R. A., Farley, M. M., Ray, S. M., Albrecht, V. S., Limbago, B. M., Hernandez, J., See, I., Satola, S. W., & Read, T. D. (2018). Invasive Methicillin-Resistant Staphylococcus aureus USA500 Strains from the U.S. Emerging Infections Program Constitute Three Geographically Distinct Lineages. mSphere, 3(3), e00571-17. https://doi.org/10.1128/mSphere.00571-17
Mairi, A., Touati, A., & Lavigne, J. P. (2020). Methicillin-Resistant Staphylococcus aureus ST80 Clone: A Systematic Review. Toxins (Basel), 12(2), 119. https://doi.org/10.3390/toxins12020119
Prevost, G., Couppie, P., Prevost, P., Gayet, S., Petiau, P., Cribier, B., Monteil, H., & Piemont, Y. (1995). Epidemiological data on Staphylococcus aureus strains producing synergohymenotropic toxins. J Med Microbiol, 42(4), 237-245. https://doi.org/10.1099/00222615-42-4-237
Saleh, M. M., Yousef, N., Shafik, S. M., & Abbas, H. A. (2022). Attenuating the virulence of the resistant superbug Staphylococcus aureus bacteria isolated from neonatal sepsis by ascorbic acid, dexamethasone, and sodium bicarbonate. BMC Microbiology, 22(1). https://doi.org/10.1186/s12866-022-02684-x
Spaan, A. N., Reyes-Robles, T., Badiou, C., Cochet, S., Boguslawski, K. M., Yoong, P., Day, C. J., de Haas, C. J., van Kessel, K. P., Vandenesch, F., Jennings, M. P., Le Van Kim, C., Colin, Y., van Strijp, J. A., Henry, T., & Torres, V. J. (2015a). Staphylococcus aureus Targets the Duffy Antigen Receptor for Chemokines (DARC) to Lyse Erythrocytes. Cell Host Microbe, 18(3), 363-370. https://doi.org/10.1016/j.chom.2015.08.001
Spaan, A. N., Schiepers, A., de Haas, C. J., van Hooijdonk, D. D., Badiou, C., Contamin, H., Vandenesch, F., Lina, G., Gerard, N. P., Gerard, C., van Kessel, K. P., Henry, T., & van Strijp, J. A. (2015b). Differential Interaction of the Staphylococcal Toxins Panton-Valentine Leukocidin and γ-Hemolysin CB with Human C5a Receptors. J Immunol, 195(3), 1034-1043. https://doi.org/10.4049/jimmunol.1500604
Spaan, A. N., Surewaard, B. G., Nijland, R., & van Strijp, J. A. (2013). Neutrophils versus Staphylococcus aureus: a biological tug of war. Annu Rev Microbiol, 67, 629-650. https://doi.org/10.1146/annurev-micro-092412-155746
Spaan, A. N., Vrieling, M., Wallet, P., Badiou, C., Reyes-Robles, T., Ohneck, E. A., Benito, Y., de Haas, C. J., Day, C. J., Jennings, M. P., Lina, G., Vandenesch, F., van Kessel, K. P., Torres, V. J., van Strijp, J. A., & Henry, T. (2014). The staphylococcal toxins γ-haemolysin AB and CB differentially target phagocytes by employing specific chemokine receptors. Nat Commun, 5, 5438. https://doi.org/10.1038/ncomms6438
Staali, L., & Colin, D. A. (2021). Bi-component HIgC/HIgB and HIgA/HIgB γ-hemolysins from S. aureus: Modulation of Ca2+ channels activity through a differential mechanism. Toxicon, 201, 74-85. https://doi.org/10.1016/j.toxicon.2021.08.007
Thelestam, M., Möllby, R., & Wadström, T. (1973). Effects of staphylococcal alpha-, beta-, delta-, and gamma-hemolysins on human diploid fibroblasts and HeLa cells: evaluation of a new quantitative as say for measuring cell damage. Infect Immun, 8(6), 938-946. https://doi.org/10.1128/iai.8.6.938-946.1973
Tong, S. Y., Davis, J. S., Eichenberger, E., Holland, T. L., & Fowler, V. G. (2015). Staphylococcus aureus infections: epidemiology, pathophysiology, clinical manifestations, and management. Clin Microbiol Rev, 28(3), 603-661. https://doi.org/10.1128/CMR.00134-14
Turner, N. A., Sharma-Kuinkel, B. K., Maskarinec, S. A., Eichenberger, E. M., Shah, P. P., Carugati, M., Holland, T. L., & Fowler, V. G. (2019). Methicillin-resistant Staphylococcus aureus: an overview of basic and clinical research. Nat Rev Microbiol, 17(4), 203-218. https://doi.org/10.1038/s41579-018-0147-4
Ye, C., Wang, Z., Hu, Y., Deng, C., Liao, L., Sun, L., & Wang, C. (2020). Systematic review and meta-analysis of the efficacy and safety of vancomycin combined with β-lactam antibiotics in the treatment of methicillin-resistant Staphylococcus aureus bloodstream infections. J Glob Antimicrob Resist, 23, 303-310. https://doi.org/10.1016/j.jgar.2020.09.024
Zimmermann-Meisse, G., Prévost, G., & Jover, E. (2017). Above and beyond C5a Receptor Targeting by Staphylococcal Leucotoxins: Retrograde Transport of Panton-Valentine Leucocidin and γ-Hemolysin. Toxins (Basel), 9(1), 41. https://doi.org/10.3390/toxins9010041

## Claims

1. A hetero-multimeric VHH antibody comprising of two or more different VHH antibody units, e.g., 2, 3 or 4 different VHH antibody units covalently fused to each other wherein at least one VHH antibody unit recognizes an *S. aureus* gamma hemolysin (Hlg).

2. The hetero-multimeric VHH antibody of claim 1, wherein the at least one VHH antibody unit recognizing an *S. aureus* gamma hemolysin (Hlg) is directed against Hlg component HlgB, HlgA or HlgC, particularly HlgB.

3. The hetero-multimeric VHH antibody of claim 1 or 2, wherein the at least one VHH antibody unit recognizing an *S. aureus* gamma hemolysin (Hlg) comprises
(a) a combination of CDR1, CDR2 and CDR3 sequences as shown in SEQ. ID NO: 6-8, 10-12, 14-16, 18-20, 22-24, 26-28, 30-32, 34-36, 38-40, 42-44, 46-48, 50-52, 54-56, 58-60, 62-64, 66-68, 70-72, 74-76, 78-80, 82-84, 86-88 or 90-92; or
(b) a combination of CDR1, CDR2 and CDR3 sequences which has an identity of at least 80%, at least 90% or at least 95% to a combination of CDR1, CDR2 and CDR3 sequences of (a).

4. The hetero-multimeric VHH antibody of any one of claims 1-3, wherein the at least one VHH antibody unit recognizing an *S. aureus* gamma hemolysin (Hlg) comprises
(a) a VHH sequence as shown in SEQ. ID NO: 5, 9, 13, 17, 21, 25, 29, 33, 37, 41, 45, 49, 53, 57, 61, 65, 69, 73, 77, 81, 85, or 89;
(b) a sequence, which has an identity of at least 80%, at least 90%, at least 95% or at least 99% to a VHH sequence of (a).

5. The hetero-multimeric VHH antibody of any one of claims 1-4 wherein the at least one VHH antibody unit recognizing an Hlg comprises anti-Hlg VHH antibody Bm29C02 having a VHH sequence as shown in SEQ. ID NO: 5 or a VHH antibody, which is a variant thereof.

6. The hetero-multimeric VHH antibody of any one of claims 1-5, which comprises
(i) at least two VHH antibody units recognizing different Hlg polypeptides selected from HlgA, HlgB, and HlgC;
(ii) at least two VHH antibody units recognizing different epitopes on the same Hlg polypeptide selected from HlgA, HlgB, or HlgC; or
(iii) a first VHH antibody unit which recognizes Hlg and a second VHH antibody unit which recognizes a different toxin or virulence factor from *S. aureus.*

7. The hetero-multimeric VHH antibody of claim 6 wherein the second VHH antibody unit recognizes *S. aureus* alpha hemolysin (Hla).

8. The hetero-multimeric VHH antibody of claim 7 wherein the second VHH antibody unit recognizing Hla comprises
(i)
(a) a combination of CDR1, CDR2 and CDR3 sequences as shown in SEQ. ID NO: 94-96, 98-100, 102-104, 106-108, 110-112, 114-116, 118-120, 122-124, 126-128, 130-132, 134-136, 138-140, 142-144, 146-148, 150-152, 154-156, 158-160, 162-164, 166-168, 170-172, 174-176, 178-180, 182-184, 186-188, 190-192 or 194-196; or
(b) a combination of CDR1, CDR2 and CDR3 sequences which has an identity of at least 80%, at least 90% or at least 95% to a combination of CDR1, CDR2 and CDR3 sequences of (a) or
(ii)
(a) a VHH sequence as shown in SEQ. ID NO: 93, 97, 101, 105, 109, 113, 117, 121, 125, 129, 133, 137, 141, 145, 149, 153, 157, 161, 165, 169, 173, 177, 181, 185, 189 or 193; or
(b) a sequence, which has an identity of at least 80%, at least 90%, at least 95% or at least 99% to a VHH sequence of (a).

9. The hetero-multimeric VHH antibody of claim 7 or 8, wherein the at least one VHH antibody unit recognizing Hla comprises the anti-Hla VHH antibody Ma7A07 having a VHH sequence as shown in SEQ. ID NO: 93 or a VHH antibody, which is a variant thereof.

10. The hetero-multimeric VHH antibody of any one of claims 1-9 wherein the VHH antibody units are covalently fused to each other via a linker, wherein the linker has a length of 5-50 amino acids, more particularly a length of 10-30 amino acids.

11. The hetero-multimeric VHH antibody of any one of claims 1-10, which is covalently or non-covalently conjugated to a heterologous moiety, and which is particularly fused to an immunoglobulin Fc fragment.

12. A set of two or more different hetero-multimeric VHH antibodies of any one of claims 1-11.

13. The hetero-multimeric VHH antibody of any one of claims 1-11 or the set of claim 12 for use in medicine, particularly for use in therapy or diagnostics, particularly for use in the prevention, treatment and/or mitigation of a condition caused by, associated with and/or accompanied by an infection with an Hlg-positive and optionally Hla-positive *S. aureus.*

14. Use of the hetero-multimeric VHH antibody of any one of claims 1-11 or the set of claim 12 for detecting Hlg and optionally Hla in a sample, wherein the sample particularly is a biological sample, e.g., a body fluid such as saliva, sputum, a lung lavage, a swab, blood, serum or plasma, a stool sample, a tissue sample, or a biopsy sample.

15. A method for the prevention, treatment and/or mitigation of a condition caused by, associated with and/or accompanied by an infection with an Hlg-positive and optionally Hla-positive *S. aureus* comprising administering an effective dose of the VHH antibody of any one of claims 1-11 or the set of claim 12 to a subject in need thereof, particularly to a human subject.
